(19) Europäisches Patentamt
European Patent Office
Office européen des brevets

(11) **EP 4 328 298 A1**

(12) **EUROPEAN PATENT APPLICATION**
published in accordance with Art. 153(4) EPC

(43) Date of publication:
**28.02.2024 Bulletin 2024/09**

(21) Application number: **22796142.2**

(22) Date of filing: **27.04.2022**

(51) International Patent Classification (IPC):
**C12M 3/00** (2006.01)    **C12M 1/32** (2006.01)
**C12M 1/12** (2006.01)

(52) Cooperative Patent Classification (CPC):
**C12M 1/12; C12M 1/32; C12M 1/34; C12M 3/00**

(86) International application number:
**PCT/KR2022/006037**

(87) International publication number:
**WO 2022/231310 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.04.2021 KR 20210054529**

(71) Applicant: **Celloid Co., Ltd.**
**Pohang-si, Gyeongsangbuk-do 37673 (KR)**

(72) Inventors:
• **KIM, Dong Sung**
**Pohang-si, Gyeongsangbuk-do 37673 (KR)**

• **LEE, Seong Jin**
**Pohang-si, Gyeongsangbuk-do 37665 (KR)**
• **KIM, Hyung Woo**
**Iksan-si, Jeollabuk-do 54630 (KR)**
• **KIM, Do Hui**
**Incheon 22000 (KR)**
• **EOM, Sung Su**
**Busan 47012 (KR)**
• **YOUN, Jae Seung**
**Pohang-si, Gyeongsangbuk-do 37836 (KR)**

(74) Representative: **BCKIP Part mbB**
**Siegfriedstraße 8**
**80803 München (DE)**

(54) **CELL CULTURE DEVICE FOR CULTURING 3D CELL AGGREGATION AND CELL CULTURE METHOD USING SAME**

(57)    The present invention relates to a culture device and culture method for culturing a 3D cell aggregation and, specifically, to an overall technique for effectively culturing a 3D cell aggregation by using a culture device including a porous microwell, a membrane, and other additive components.

Fig. 1

**Description**

**Technical Field**

The present invention relates to a culture device and culture method for culturing a 3D cell aggregation and, specifically, to an overall technique for effectively culturing a 3D cell aggregation by using a culture device including a porous microwell, a membrane, and other additive components.

**Background Art**

Actual cells in the body have a three-dimensional shape and interact with cellular microenvironment in three dimensions. Cells cultured *in vitro* as two-dimensional monolayers rather than three dimensions largely lack morphological similarity to *in vivo* cells, and cells cultured in three dimensions may show phenomena similar to those of actual *in vivo* cells when used for drug screening and cell therapy.

In order to overcome the limitations of the above-described two-dimensional monolayer cell culture, the demand for a microwell platform capable of culturing and differentiating three-dimensional (3D) cell aggregates has increased worldwide.

Meanwhile, conventional microwells do not have a porous structure or are formed from plastic, etc., and thus have limitations in forming 3D cell aggregates, and even if 3D cell aggregates are formed, there is a problem in that the process of detaching the 3D cell aggregates from the microwell platform is not easy. In addition, conventionally proposed culture environments have a problem in that they rely only on passive diffusion for waste discharge and nutrient supply during culture, which limits the growth and maturation of 3D cell aggregates.

The present invention has been made in view of the above-described limitations and problems, and relates to a culture device capable of culturing three-dimensional cell aggregates more effectively than a conventional art and a culture method using the same.

Additionally, in addition to solving the above technical problems, the present invention was invented to provide additional technical elements that cannot be easily invented by those skilled in the art.

**DISCLOSURE**

**Technical Problem**

An object of the present invention is to provide an environment in which three-dimensional cell aggregates, more specifically, three-dimensional cell spheroids, may be formed.

In addition, another object of the present invention is to induce the formation of three-dimensional cell aggregates at cell culture temperature and maintain the characteristics of easy culture, and to enable large quantities of three-dimensional cell aggregates to be simultaneously detached from the microwell plate at room temperature.

In addition, another object of the present invention is to enable stable three-dimensional cell culture and flow control at the bottom of the porous microwell so that waste products can be discharged efficiently during cell culture and nutrients can be smoothly supplied to the bottom of the cells.

In addition, another object of the present invention is to minimize the loss of cells and three-dimensional cell aggregates caused by pipetting on the cell culture surface, which is the upper surface of the microwell, for repeated cell culture medium replacement, which is widely used in the past. Additionally, the purpose is to provide an environment that can continuously provide a uniform surrounding environment for cell aggregates.

**Technical Solution**

A 3D cell culture device according to the present invention comprises: an upper chamber, comprising porous microwell comprising an opening, a porous membrane, and cell culture space for accommodating a culture medium; a lower chamber, comprising a space in which the upper chamber is disposed; wherein fluid flowing into the upper part of the upper chamber passes through the porous microwell in the upper chamber and flows into the lower chamber, and wherein the fluid in the lower chamber is discharged and the fluid flows.

According to another embodiment of the present invention, a method of culturing 3D cell aggregate using a 3D culture device according to the 3D cell culture device, the method comprises steps of: seeding cells on the porous membrane of the upper chamber; introducing a cell culture medium into the upper portion of the upper chamber through an opening; and discharging the cell culture medium, that permeated the porous microwell in the upper chamber and flowed into the lower chamber, from the lower chamber.

**Advantageous Effects**

According to the present invention, it is possible to provide an environment in which three-dimensional cell aggregates or cell spheroids may be effectively formed, so that cells settled in a certain area may more smoothly proliferate and differentiate in three dimensions.

In addition, according to the present invention, it is possible to implement a microwell plate with a surface structure having a surface roughness that is convenient for culturing and harvesting cells through temperature changes, which has the effect of enabling mass production and harvesting of three-dimensional cell aggregates.

In addition, according to the present invention, it is possible to create a culture environment that efficiently removes waste products formed during cell culture and smoothly supplies nutrients to the bottom of the three-dimensional cells without adversely affecting the settlement, proliferation, and differentiation of cells.

In addition, according to the present invention, it is possible to significantly reduce the possibility of loss of cells and cell aggregates during the process of culturing cells, as well as to continuously provide a uniform microenvironment around the cell aggregates. Furthermore, the level of the culture medium reservoir can be set to correspond to the level of the culture medium in the lower chamber, so that the level of the culture medium can be kept constant during the cultivation process.

In addition, by using porous microwells with very excellent hydraulic conductivity, the hydraulic pressure applied to the porous microwells and the three-dimensional cell aggregates being cultured within them can be minimized, thereby minimizing unnecessary stimulation other than the flow.

**Brief Description of Drawings**

FIG. 1 shows a cell culture vessel according to an exemplary embodiment of the present invention.

FIG. 2 is a cross-sectional diagram of one side of the cell culture vessel according to an exemplary embodiment of the present invention.

FIG. 3 shows a plate 30 according to an exemplary embodiment of the present invention.

FIG. 4 shows an enlarged diagram of the periphery of one opening 31 in FIG. 2.

FIG. 5 shows a state in which cells proliferate in a cell culture vessel according to another exemplary embodiment of the present invention.

FIG. 6 shows a body 10 and a fastening part 40 of a cell culture vessel according to another exemplary embodiment of the present invention.

FIG. 7 shows a sequence of the method for manufacturing a cell culture vessel according to another exemplary embodiment of the present invention.

FIG. 8 shows an example of S10 or S100 of the method for manufacturing a cell culture vessel according to another exemplary embodiment of the present invention.

FIG. 9 shows an example of S20 or S200 of the method for manufacturing a cell culture vessel according to another exemplary embodiment of the present invention.

FIG. 10 shows images of a membrane 20 formed by the method for manufacturing a cell culture vessel according to an exemplary embodiment of the present invention.

FIG. 11 shows top images of the upper side of the upper chamber of a cell culture vessel manufactured according to another embodiment of the present invention. FIG. 11B is an enlarged view of one of the plurality of porous microwells shown in FIG. 11A.

FIG. 12 shows temperature- and time-dependent changes in the surface roughness of a cell culture layer.

FIG. 13A is an enlarged view of a porous microwell, and FIG. 13B shows that a cell culture layer comprising polyisopropylacrylamide has been formed on the upper surface of the porous microwell.

FIG. 14A shows an image taken in the process of culturing three-dimensional cell aggregates. FIG. 14B shows cell aggregates detached from a well plate. FIG. 14C shows an image of the well plate after detachment of the cell aggregates.

FIG. 15(a) is a view illustrating a three-dimensional cell culture device according to one embodiment of the present invention, FIG. 15(b) is a view illustrating a through portion formed adjacent to a porous microwell in the upper chamber of the three-dimensional cell culture device according to the present invention, and FIG. 15(c) shows actual images of the three-dimensional cell culture device.

FIG. 16(a) shows a comparison of the albumin expression levels measured in Example 2 of the present invention and Comparative Example 2, and FIG. 16(b) shows a comparison of the FITC-dextran concentrations measured in Example 3 of the present invention and Comparative Example 4.

FIG. 17 shows the results of visually observing a state in which wastes have been removed over time, according to Example 3 of the present invention.

FIG. 18 schematically shows that wastes on the upper surface of the porous microwell are removed by the fluid flow in the lower chamber and the porous microwell according to the present invention, and schematically shows that a culture medium is diffused into the porous microwell.

FIG. 19 shows a cell culture device according to another embodiment of the present invention. Specifically, FIG. 19(a) shows the overall appearance of the cell culture device, FIG. 19(b) is an enlarged view of a combination of one upper chamber and one lower chamber, and FIG. 19(c) is a cross-sectional view of a cell culture device comprising a culture medium reservoir connected to an outlet.

FIG. 20 shows the results of simulating the flow of a culture medium that passing through a porous membrane in the cell culture device according to the present invention.

FIG. 21 shows micrographs of a porous microwell configured to comprise a porous membrane having concave portions. FIG. 21(a) is a 4x magnified image, and FIG. 21(b) is a 220x magnified image. FIG. 21(c) show images of another type of porous microwell comprising a porous membrane having concave portions. Specifically, FIG. 21(c)a) is an image taken by a DSLR camera, and FIGS. 21(c)b) and (c)c) are 4x and 20x magnified images, respectively.

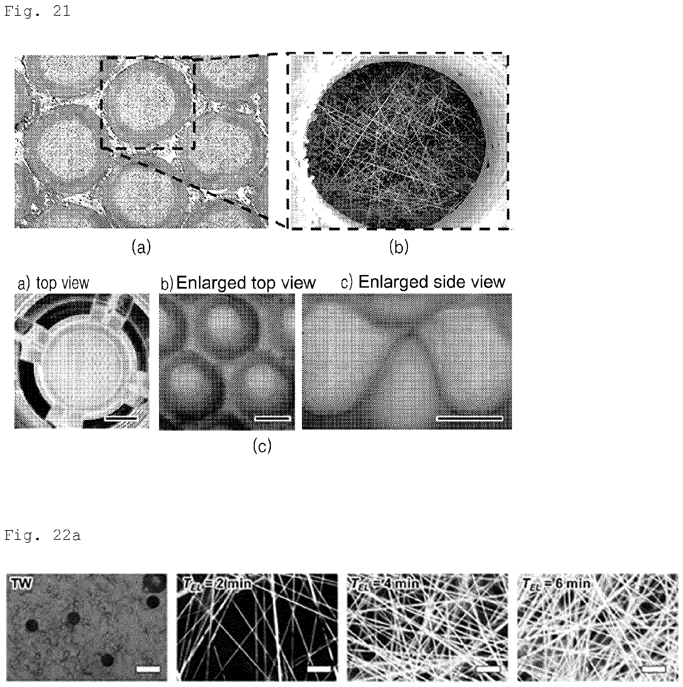

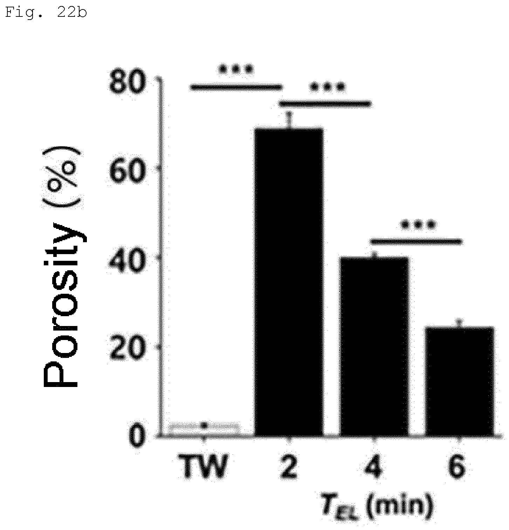

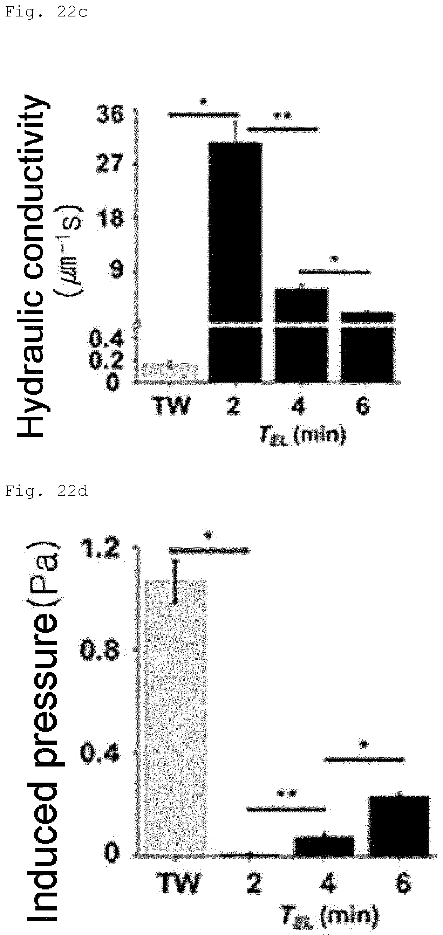

FIG. 22 shows changes in the structure and properties of the nanofibrous networks of porous membranes fabricated at different electrospinning times. Transwell insert (Corning, USA) with a pore size of 8 $\mu$m, which is a commercially available porous membrane, is also shown for reference (the very left, TW). FIG. 22(a) shows 20x magnified microscopic images of the commercially available membrane and nanofibrous network structures fabricated at different electrospinning times. FIGS. 22(b), 22(c) and 22(d) show the porosities (FIG. 22(b)), hydraulic conductivities (FIG. 22(c)) and induced pressures (FIG. 22(d)) of the commercially available membrane and the nanofibrous network structures fabricated at different electrospinning times.

FIG. 23 shows experimental results obtained by comparing the degree of loss of cell aggregates between the case in which the cell culture device according to the present invention is used and the case in which cells are cultured through a conventional method of replacing cell culture medium by pipetting.

FIG. 24 shows the results of numerical simulation performed to compare the nutrient (glucose) concentration around cell aggregates between the case in which the cell culture device according to the present invention is used and the case in which cells are cultured through a conventional method of replacing cell culture medium by pipetting.

FIG. 25 shows a state in which a fluid present in a cell culture device is smoothly replaced with a fresh fluid while the level of the culture medium in the lower chamber is kept constant.

FIG. 26 shows a method of manufacturing a porous microwell.

FIG. 27 shows a lower chamber (a) used in the cell culture device according to the present invention, and an upper chamber (b) inserted into the lower chamber.

FIG. 28 illustrates a method of manufacturing a porous microwell using a compression process.

FIG. 29 shows a process of forming a fluid flow by allowing fluid to be discharged through the gap region between the upper chamber and the lower chamber. Specifically, FIG. 29(a) shows photographs, FIG. 29(b) is a schematic image, and FIG. 29(c) represents the flow rate of the fluid formed.

**Mode for Invention**

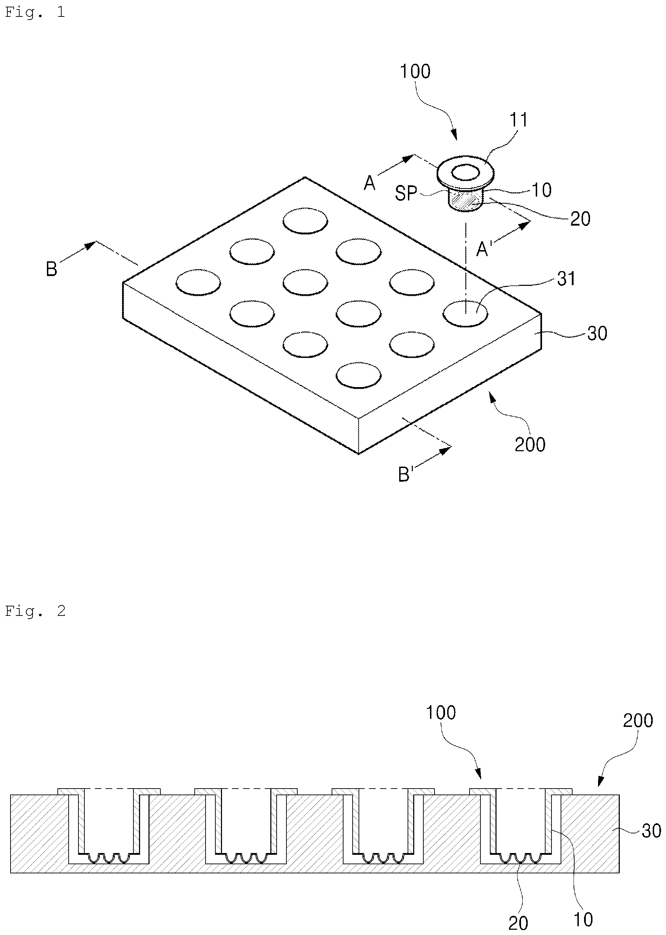

First, a cell culture vessel, which is a basic configuration of a cell culture device, and a method for manufacturing the same, will be described with reference to FIGS. 1 to 10.

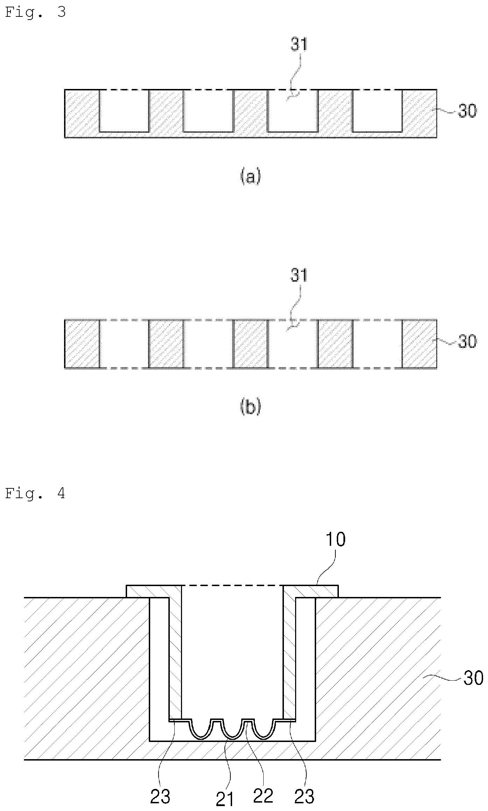

FIG. 1 shows a cell culture vessel according to an exemplary embodiment of the present invention, and FIG. 2 is a cross-sectional diagram of one side of the cell culture vessel according to an exemplary embodiment of the present invention. In this case, FIG. 2 shows a cross section of one side cutting A and A' in FIG. 1. FIG. 3 shows a plate 30 according to an exemplary embodiment of the present invention.

The cell culture vessel according to an exemplary embodiment of the present invention may include a body 10 and a membrane 20, as illustrated in FIGS. 1 and 2, and may further include a plate 30. However, hereinafter, for convenience of description, the plate 30 will be described first, and then, the body 10 and the membrane 20 will be described in order.

The plate 30 has one or more openings 31 having an opening shape, and the body 10 may be inserted and mounted into the opening 31. In this case, the opening 31 may have an open shape with a lower portion closed and an upper portion open, as illustrated in FIG. 3(a), or a penetrating shape penetrating the plate 30, as illustrated in FIG. 3(b). When the opening 31 has a penetrating shape, the cell culture vessel according to an exemplary embodiment of the present invention may further include a cover container having an accommodating space with an open upper portion and mounting the plate 30 in the accommodating space.

In particular, when a plurality of openings 31 are provided in the plate 30, since each opening 31 is arranged to be separated from each other, the cell culture vessel according to an exemplary embodiment of the present invention may block the influence between samples of each opening 31 during cell culture, and accordingly, multiple independent experimental data may be derived using one plate 30.

As illustrated in FIGS. 1 and 2, the body 10 includes a spacer formed at one end to have a side wall, an inlet 11 formed at the other end, and a penetrating part penetrating the spacer and the inlet 11, respectively. For reference, the spacer refers to a part corresponding to the side or side wall of the body 10, and is indicated by the reference numeral SP in the drawing. As can be seen in the drawing, the outer surface of the spacer faces the inner surface of the opening 31, but it can be confirmed that a predetermined gap or space exists between the spacer and the inner surface of the opening.

The spacer of the body 10 may be inserted into the opening 31 of the plate 30. In this case, the body 10 may include one insertion part or may include a plurality of insertion parts. That is, when including one insertion part, the body 10 may be inserted into one opening 31 of the corresponding insertion part. In addition, when a plurality of insertion parts are included, each insertion part may be inserted corresponding to a plurality of openings 31 in the body 10. In this case, when a plurality of insertion parts are included, the body 10 has a structure in which each insertion part is connected to each other, and each insertion part may be inserted corresponding to each opening 31.

While FIGS. 1 to 6 illustrate a body 10 including one insertion part, the present invention is not limited thereto, and the contents of the present invention may certainly be applied even when the body 10 includes a plurality of spacers and penetrating parts.

When the spacer of the body 10 is inserted into the opening 31, the spacer of the body 10 is located at the bottom, and the inlet 11 of the body 10 is located at the top. The spacer of the body 10 may have a vertical shape having a constant width from one end to the other end, a funnel shape in which the width gradually expands from one end to the other end, or a form in which a vertical form and a funnel form are combined. The penetrating part of the body 10 may be formed in various shapes such as a circular shape, a polygonal cross section, or the like, and various sizes thereof may be formed.

FIG. 4 shows an enlarged diagram of the periphery of one opening 31. In particular, when the spacer of the body 10 is mounted on the opening 31 of the plate 30, as illustrated in FIG. 4, it is preferable that the inlet 11 of the body 10 has a cross section that is wider than the inlet of the spacer and the opening 31 of the body, in order that one end of the spacer of the body 10 is located at a certain distance from the bottom surface of a cell culture vessel. Accordingly, in the space between the spacer of the body 10 and the bottom surface of the cell culture vessel, a passage for a fluid having nutrients for supplying the cells may be formed. In this case, the bottom surface of the cell culture vessel may be the bottom surface of the opening in the case of an open plate, and may be the bottom surface of the cover container in the case of a penetrating plate.

The membrane 20 is a layer that provides a cell culture surface on which cells are cultured, and is employed in the penetrating part on the side of the spacer of the body 10. For example, the membrane 20 may be formed through electrospinning to cover one end of the spacer of the body 10. In this case, the membrane 20 may be formed by randomly intertwining a plurality of polymer nanofibers, or may be formed by molding a polymer synthetic resin. For example, each polymer nanofiber may have a diameter of 1 nm or more to less than 1,000 nm. As it is made of a plurality of polymer nanofibers, the membrane 20 has a structure similar to that of the basement membrane in a living body, thereby providing a blood flow environment in the living body.

For example, the polymer nanofiber or polymer synthetic resin may include at least one or more of a thermoplastic resin, a thermosetting resin, an elastomer, and a biopolymer. For example, polymer nanofibers or synthetic resins may include at least one or more of polycaprolactone, polyurethane, polyvinylidene fluoride (PVDF), polystyrene, collagen, gelatin, and chitosan.

The membrane 20 may include a porous microwell 21, a connection part 22, and a fixing part 23. In this case, the microwell 21, the connection part 22, and the fixing part 23 have a structure in which these are connected to each other by intertwining a plurality of polymer nanofibers.

The microwell 21 is a region that acts as a cell culture surface, and is formed to be indented in a downward direction. That is, by this indented shape, cells are easily seated in the microwell 21 and may be stably proliferated in the microwell 21 regardless of the movement of a fluid. In this case, at least one of the microwells 21 is located in a region formed by the penetrating part of the body 10. That is, when viewed from the top or bottom, the microwell 21 is smaller in size than the penetrating part of the body 10 and is included in the region formed by the penetrating part.

That is, as the microwell 21, which is a cell culture surface, is formed in an indented shape, the membrane 20 may attach the cells to the cell culture surface more intensively and stably and increase the area of the cell culture surface such that it may improve the adhesion efficiency of cells. In addition, unlike conventional cell culture vessels having a cell culture surface of conventionally flat shapes, the body 10 of the cell culture vessel according to an exemplary embodiment of the present invention may form a three-dimensional cell spheroid by providing a cell culture surface in a three-dimensional shape to culture cells in a three-dimensional structure environment as in the living body. However, when a plurality of microwells 21 are located within a region formed by the penetrating part of the body 10, the spacer of the body 10 has a plurality of cell culture surfaces such that cell adhesion efficiency may be further improved.

The connection part 22 is a region formed around the microwells 21 to connect between the microwells 21 and may have a flat shape. In particular, the connection part 22 may be thicker than the microwell 21. This corresponds to a region in which the microwell 21 extends in a lower indented shape among the membranes 20 by an embossing process to be described below such that it becomes thinner than the original, whereas the connection part 22 corresponds to a region that is not extended such that the original thickness is maintained.

The fixing part 23 is an area fixed to the edge of one end of the spacer of the body 10. In particular, the fixing part 23 may have a thinner thickness and a lower density than the connection part 22. This is because the membrane 20 may be formed through electrospinning using an electrolyte solution. That is, since the microwell 21 and the connection part 22 correspond to a region generated at a location where the electrolyte solution is contained during electrospinning, a greater number of polymer nanofibers are formed than the fixing part 23, and thus, it may be formed with a higher density and thicker thickness than the fixing part 23.

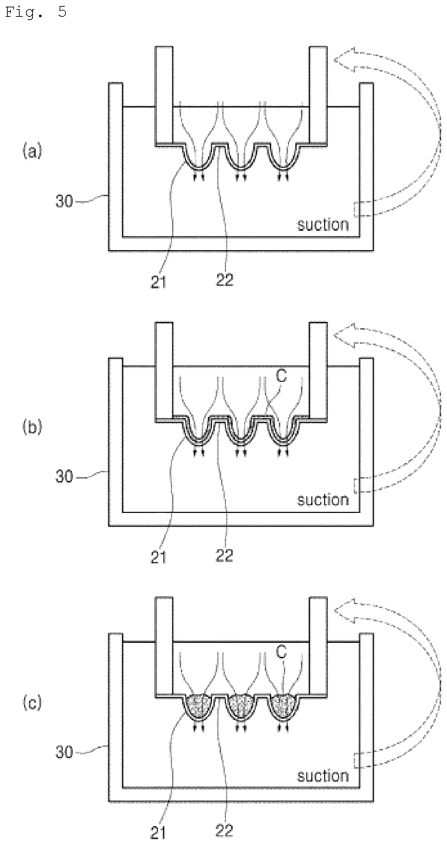

FIG. 5 shows a state in which cells proliferate in a cell culture vessel according to another exemplary embodiment of the present invention. That is, FIGS. 5(a), 5(b), and 5(c) sequentially show a state in which cells proliferate over time under a fluid concentration phenomenon.

Meanwhile, the membrane 20 includes a plurality of pores formed in a region between the polymernanofibers. In this case, the pores may have a size of several um to tens of um. That is, the membrane 20 may act as a selective permeable membrane that does not allow single cells to permeate but selectively permeates other materials due to the pores, and thus may serve as a material transfer barrier and passage.

The first porosity formed by the first pores formed in the microwell 21 and the second porosity formed by the second pores formed in the connection part 22 may be different from each other. In this case, the porosity may be a ratio of the pore area existing in the unit area. In particular, the first porosity may be greater than the second porosity. This is because the region of the membrane 20 corresponding to the microwell 21 is stretched into a lower indented shape by the embossing process, and a phenomenon occurs in which a number of portions blocked by the intertwined polymer nanofibers are opened or the area of the already opened portion is widened. However, these two porosities are described in order to have a difference between the first porosity and the second porosity, but the present invention is not limited to having only two types of these porosities. That is, the present invention may have various porosities in addition to the first porosity and the second porosity.

According to the difference in such porosities, as illustrated in FIG. 5, while a fluid concentration phenomenon occurs in a region around the microwell 21, cell proliferation in the microwell 21 may occur more actively. This is because as the region has a high porosity, the material permeability is higher according to Darcy's equation.

For cell culture, a fluid (e.g., a mixture of cell culture solution, distilled water, PBS solution, etc.) is filled in the opening 31 of the plate 30, and this fluid should be periodically replaced by using a spuit or the like. In this case, the fluid may be replaced by a method in which the fluid is suctioned and discharged from the outside of the body 10 and a new fluid is supplied to the inside of the body 10 at the same time. This is because when the fluid is suctioned and discharged from the inside of the body 10, there is a risk that the cells that are seated in the microwell 21 and proliferating and differentiating may be adversely affected or the corresponding cells may be discharged together.

In the above-described fluid replacement process, the fluid filled in the accommodating space accommodating the microwell 21 passes from the top of the microwell 21 and the connection part 22 to the bottom. In this case, since the microwell 21 has a greater porosity than the connection part 22, as illustrated in FIG. 5, more fluid permeates through the microwell 21 compared to the connection part 22. Accordingly, a phenomenon in which the fluid moves more intensively, that is, a fluid concentration phenomenon, occurs in the vicinity of the microwell 21 than in the vicinity of the connection part 22.

When such a fluid concentration phenomenon occurs, oxygen and nutrients contained in the fluid may be more smoothly supplied to cells proliferating and differentiating within the microwell 21, and thus, cell proliferation and differentiation may be further promoted. In addition, due to this fluid concentration phenomenon, a phenomenon in which cells are collected into the microwell 21 also occurs. That is, a plurality of regions having a difference in porosity are provided, but as the microwell 21, which is a cell culture surface among the corresponding regions, is formed to have a higher porosity than the connection part 22, the membrane 20 may increase the efficiency of proliferation and differentiation of cells in the microwell 21.

When a plurality of openings 31 are provided in the plate 30, the cell culture vessel according to the present invention may derive multiple independent experimental data tested in a three-dimensional structure environment, such as in the living body, using one plate 30.

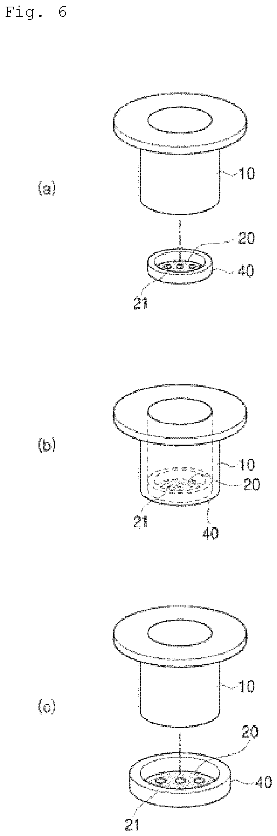

FIG. 6 shows a body 10 and a fastening part 40 of a cell culture vessel according to another exemplary embodiment of the present invention.

Meanwhile, as illustrated in FIG. 6, the cell culture vessel according to another exemplary embodiment of the present invention may further include a fastening part 40 having one end and the other end passing through and fastened to one end of the spacer of the body 10.

In this case, the fastening part 40 may include one penetrating part or may include a plurality of penetrating parts. That is, in the case of including one penetrating part, the fastening part 40 may be fastened to each spacer of the body 10 and may be formed in a ring shape. In addition, when a plurality of penetrating parts are included, the fastening part 40 may be fastened with each penetrating part corresponding to each spacer of the body 10. While FIG. 6 illustrates a case where the fastening part 40 includes one penetrating part, the present invention is not limited thereto, and the contents of the present invention may certainly be applied even when the fastening portion 40 includes a plurality of penetrating parts.

When the fastening part 40 is further included, the membrane 20 is not provided at one end of the spacer of the body 10, and the membrane 20 may be provided at one end of the fastening part 40, or the membrane 20 may be provided together at one end of the spacer of the body 10 and one end of the fastening part 40. When the fastening part 40 is fastened to the spacer of the body 10, the penetrating part of the fastening part 40 and the penetrating part of the body 10 are connected to each other.

The fastening part 40 may be provided in a form that is detachable (mounted and separated) at one end of the spacer of the body 10. For example, a screw thread may be formed inside or outside the fastening part 40, and a screw thread corresponding to the screw thread of the fastening part 40 may be formed outside or inside one end of the body. In addition, as illustrated in FIG. 6(b), the fastening part 40 may be fitted and coupled to the inner circumferential surface of the penetrating part of one end of the spacer of the body 10, or as illustrated in FIG. 6(c), it may be fitted and coupled to the outer circumferential surface of one end of the spacer the body 10.

The membrane 20 provided at one end of the fastening part 40 is the same except that the spacer of the body 10 is replaced with the fastening part 40 in the membrane 20 provided at one end of the spacer of the body 10 described above. That is, the position of the membrane 20 provided at one end of the fastening part 40 is only changed from one end of the spacer of the body 10 to one end of the fastening part 40. Accordingly, detailed descriptions of the membrane 20 provided at one end of the fastening part 40 will be omitted below, and it will be replaced with a description of the membrane 20 provided at one end of the spacer of the body 10 described above.

Hereinafter, the method for manufacturing a cell culture vessel according to an exemplary embodiment of the present invention will be described. The method for manufacturing such a cell culture vessel includes a method for manufacturing a microwell 21 or a membrane 20.

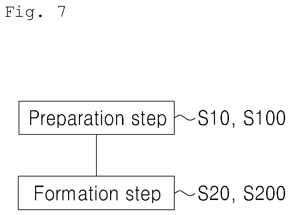

FIG. 7 shows a sequence of the method for manufacturing a cell culture vessel according to another exemplary embodiment of the present invention.

As illustrated in FIG. 7, the method for manufacturing a cell culture vessel according to an exemplary embodiment of the present invention includes preparation steps S10, S100 and formation steps S20, S200. In this case, S10 and S20 are the manufacturing methods of the body 10 and the membrane 20 described above, and S100 and S200 are the manufacturing methods of the body 10, the membrane 20, and the fastening part 40 described above.

S10 is a step of preparing the body 10 and the membrane 20. In addition, S100 is a step of preparing the body 10, the membrane 20, and the fastening part 40. In this case, since the body 10, the membrane 20, and the fastening part 40 are the same as those described above according to FIGS. 1 to 6, descriptions thereof will be omitted below. However, the membrane 20 may be formed through an electrospinning method using an electrolyte solution, and the electrospinning method using an electrolyte solution will be described below.

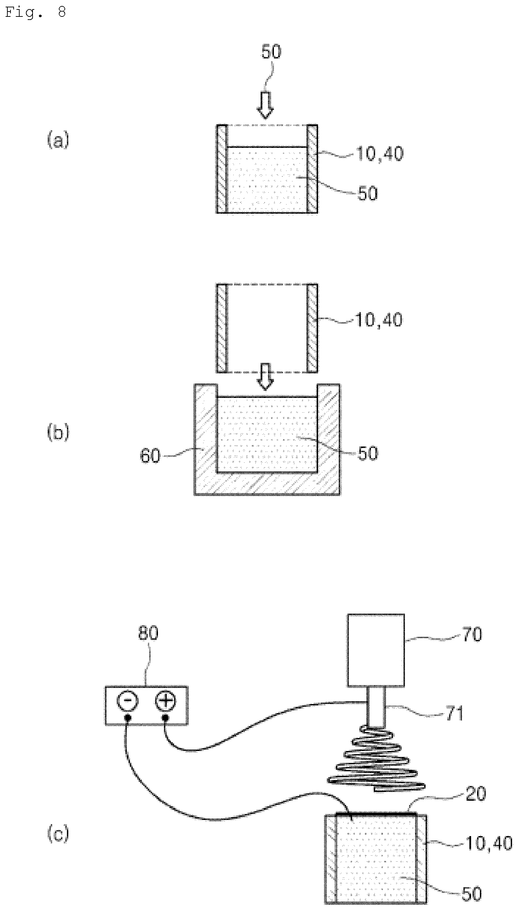

FIG. 8 shows an example of S10 or S100 of the method for manufacturing a cell culture vessel according to another exemplary embodiment of the present invention.

The electrospinning method using an electrolyte solution is to form the membrane 20 so as to cover one end of the spacer of the body 10 or the fastening part 40, and may be performed inside a chamber. In this case, the chamber is a space in which work is performed, and when the membrane 20 is formed, leakage of the polymer solution to the outside may be prevented. Hereinafter, the electrospinning method using an electrolyte solution forming the membrane 20 at one end of the spacer of the body 10 is referred to as "a first electrospinning method", and the electrospinning method using an electrolyte solution forming the membrane 20 at one end of the fastening portion 40 is referred to as "a second electrospinning method." First, the first electrospinning method will be described.

The first electrospinning method may sequentially include an electrolyte filling step, a voltage application step, and a membrane formation step.

That is, as illustrated in FIG. 8(a), the electrolyte filling step is a step of filling an electrolyte solution 50 in the spacer of the body 10 formed such that one end and the other end pass through. In this case, the body 10 is disposed such that one end of the spacer faces upward and the other end is blocked. Afterwards, the electrolyte solution 50 is filled into one end of the spacer of the body 10. In this case, a stopper is provided to close the inlet 11 of the body 10, and the corresponding stopper may be provided with an electrode for applying a voltage to the electrolyte solution 50. That is, the electrode is formed to pass through the stopper, and may be connected to the electrolyte solution 50 filled in the spacer of the body 10.

Alternatively, as illustrated in FIG. 8(b), in the electrolyte filling step, the spacer of the body 10 may be disposed in the electrolyte container 60 filled with the electrolyte solution 50, but the electrolyte solution 50 may be filled in the penetrating part of the body 10 by disposing one end of the spacer of the body 10 towards the top. When the spacer of the body 10 is disposed in the accommodating space of the electrolyte container 60, a pressure to press the surface of the electrolyte solution 50 is generated while the spacer of the body 10 is in contact with the surface of the electrolyte solution 50, and by this pressure, the electrolyte solution 50 is filled in the spacer of the body 10. In this case, in order that the pressure on the surface of the electrolyte solution 50 may be better generated, the accommodating space of the electrolyte container 60 may be formed to match the shape of the spacer of the body 10.

Since the electrolyte solution 50 has conductivity, when a voltage is applied in the voltage application step, it becomes (-) charge, attracting particles with (+) charge by electrical attraction, and accordingly, particles with (+) charge may be accumulated on top of the electrolyte. The electrolyte solution 50 is classified into a strong electrolyte and a weak electrolyte according to the degree of dissociation. The degree of dissociation is different depending on the solvent.

For example, as the electrolyte solution 50, a solution obtained by nixing potassium chloride and distilled water at a ratio of 3% mol may be used. In addition, materials dissolved in water or an organic solvent (ethanol and methanol) and exhibit electrical conductivity higher than 1 mS/cm may be used as the electrolyte solution 50. In addition, materials at concentrations dissolved in water and having a relative dielectric constant higher than 80 F/m may be used as the electrolyte solution 50.

Afterwards, as illustrated in FIG. 8(c), the voltage application step is a step of applying a voltage between an electrolyte solution 50 and a metal needle 71 of an electrospinning machine 70. In this case, the voltage is supplied through a power supply, and the structure of the membrane 20 formed in the membrane formation step formed in accordance with changes in the intensity of the applied voltage may be changed.

That is, an electric field is formed between the electrolyte solution 50 and the metal needle 71 of the electrospinning machine 70, and if the strength of the electric field formed at this time is too low, the polymer solution is not continuously discharged. Thus, not only is it difficult to manufacture polymer nanofibers having a uniform thickness, but also it may be difficult for the manufactured polymer nanofibers to be smoothly focused on the electrolyte solution 50. Conversely, when the strength of the electric field is too high, it may be difficult to have a normal shape because the polymer fibers are not accurately seated on the upper surface of the electrolyte solution 50. In consideration of this content, the strength of the voltage applied to the electrolyte solution 50 and the metal needle 71 of the electrospinning machine 70 may range from 5 kV to 30 kV.

A negative (-) voltage may be applied to the electrolyte solution 50, and a positive (+) voltage may be applied to the metal needle 71. Accordingly, the electrolyte solution 50 has a negative (-) charge, and the polymer solution spun in the membrane formation step has a positive (+) charge.

Afterwards, as illustrated in FIG. 8(c), the membrane formation step is a step in which a polymer solution is spun to the spacer of the body 10 through the electrospinning machine 70 while a voltage is applied to form the membrane 20. In this case, the membrane 20 is formed in a network shape in which a plurality of polymer nanofibers are randomly intertwined due to the high degree of freedom of the electrolyte solution 50.

Meanwhile, the electrospinning machine 70 is a device for supplying a polymer solution. That is, the electrospinning machine 70 may store the polymer solution to have an appropriate viscosity for electrospinning, and then discharge the polymer solution through the metal needle 71. In this case, the discharged polymer solution may be scattered and cured at the same time to form polymer nanofibers.

The metal needle 71 is a configuration to discharge a polymer solution. As it is made of a metal material, the metal needle 71 is easily connected to the power supply, and it is possible to improve the charge charging efficiency of the polymer solution discharged when a voltage is applied from the power supply. In particular, the metal needle 71 is located at an upper portion spaced apart from the spacer of the body 10, but while the discharging end thereof is disposed to face the spacer of the body 10, the polymer solution may be spun.

For example, the electrospinning machine 70 may be composed of a syringe, a syringe pump, and a metal needle 71. That is, the polymer solution may be put into a syringe, and the polymer solution may be discharged into the air by the metal needle 71 through the power of the syringe pump. In this case, the metal needle 71 may use a 23 Gauge needle, but the size of may vary depending on the polymer solution. In particular, the polymer solution may be spun at a discharge rate of 0.01 mL/h to 3 mL/h such that polymer fibers may be placed on the surface of the electrolyte solution 50 while maintaining the surface shape of the electrolyte solution 50.

When the polymer solution is spun in the above-described voltage application range (5 kV to 30 kV) and discharge rate range (0.01 mL/h to 3 mL/h), the polymer nanofibers may be formed to have a diameter of 10 nm to 900 nm.

For example, as the polymer solution, a solution having a concentration of 5% to 25% in which polycaprolactone is mixed with a solution of chloroform and methanol mixed at a mass ratio of 1:1 may be used. In addition, after mixing acetone and dimethylformamide at a volume ratio of 3:7, a solution having a concentration of 25% to 30% in which polvvinylidene fluoride (PVDF) is mixed may be used as a polymer solution. Other than the above, a polymer solution may be prepared using polystyrene, polycarbonate, a collagen/polycarbonate blending solution, gelatin, and the like.

In particular, in the membrane formation step, the electrical attraction generated between the penetrating part on the side of the spacer of the body 10 filled with the electrolyte solution 50 and the polymer solution is constant, but it is larger than the electric attraction generated between the rim of the spacer of the body 10 and the polymer solution. Accordingly, the region of the membrane 20 (hereinafter, referred to as "a penetrating part region") accumulated in the penetrating part on the side of the spacer of the body 10 filled with the electrolyte solution 50 is constant, but it has a relatively large density and thickness.

On the other hand, the size of the electrical attraction generated between the rim of the spacer of the body 10 and the polymer solution is smaller than the size of the electrical attraction generated between the penetrating part of the spacer of the body 10 and the polymer solution, and it becomes smaller as it is further away from the penetrating part of the spacer of the body 10. Accordingly, the fixing part 23, which is the membrane 20 accumulated on the rim of the spacer of the body 10, is not constant, but has a relatively small density and thickness.

The membrane formation step may further include controlling at least one of thickness, porosity, and transparency of the membrane 20 formed at one end of the spacer of the body 10 by adjusting the spinning time of the electrospinning machine 70. That is, as the spinning time of the electrospinning machine 70 increases, the amount of polymer nanofibers to be accumulated increases. Accordingly, as the thickness of the membrane 20 formed on one end of the spacer of the body 10 increases, the porosity and transparency thereof decrease.

In addition, the membrane formation step may further include adjusting the diameter of the polymer nanofibers of the membrane 20 formed by adjusting the concentration of the polymer solution. That is, as the concentration of the polymer solution increases, the viscosity thereof increases such that the diameter of the polymer nanofibers of the membrane 20 formed at one end of the spacer of the body 10 increases.

Next, a second electrospinning method will be described.

The second electrospinning method, like the first electrospinning method, includes an electrolyte filling step, a voltage application step, and a met brane formation step, and may further include a fastening part-fastening step. In this case, the electrolyte filling step, the voltage application step, and the membrane formation step are the same as described above except that the spacer of the body 10 is replaced with the fastening part 40 in the first electrospinning method. Accordingly, detailed descriptions of the electrolyte filling step, voltage application step, and membrane formation step of the second electrospinning method are omitted below, and these will be replaced with descriptions of the electrolyte filling step, voltage application step, and membrane formation step of the first electrolyte electrospinning method described above.

That is, in the second electrospinning method, the membrane 20 may be formed at one end of the fastening part 40 through an electrolyte filling step, a voltage application step, and a membrane formation step. Afterwards, the fastening part-fastening step is a step of fastening the fastening part 40 on which the membrane 20 is formed at one end of the spacer of the body 10 formed such that one end and the other end pass through. For example, the fastening part-fastening step may be performed by a transfer device that transfers the spacer of the body 10 or the fastening part 40 to fasten the spacer of the body 10 and the fastening part 40.

However, the first electrospinning method and the second electrospinning method may further include a step of cutting the membrane 20 formed according to the shape of the spacer of the body 10 or the fastening part 40, after forming the membrane 20.

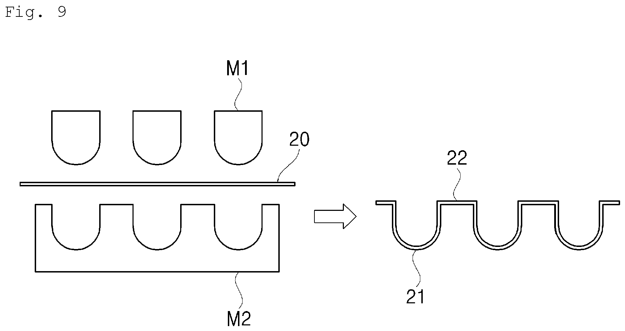

FIG. 9 shows an example of S20 or S200 of the method for manufacturing a cell culture vessel according to another exemplary embodiment of the present invention.

S20 is a step of performing an embossing process on the membrane 20 formed on the spacer of the body 10. In addition, S200 is a step of performing an embossing process on the membrane 20 formed on the fastening part 40.

That is, in S20 or S200, as illustrated in FIG. 9, an embossing process is performed on the membrane 20 prepared in S10 or S100 using a mold M in which the pattern of the microwell 21 is formed. In this case, the embossing process is a process of forming a pattern corresponding to the pattern of the mold M on the membrane 20 by pressing the membrane 20 with the mold M. That is, as a result of the embossing process, the microwell 21 and the connection part 22 may be formed in the membrane 20.

The embossing process may be a hot embossing process in which the membrane 20 is pressed with the heated mold M, but is not limited thereto. However, in the case of using a hot embossing process, the result of S20 may be derived more quickly.

The mold M may include a first mold M1 whose lower portion protrudes according to the pattern of the microwell 21 and a second mold M2 whose upper portion is indented according to the pattern of the microwell 21. That is, the membrane 20 is placed between the first mold M1 and the second mold M2, and the microwell 21 and the connection part 22 may be formed in the membrane 20 by pressing and combining the first mold M1 and the second mold M2 and then separating. When combined, the protruding portion of the first mold M1 contacts one surface of the membrane 20, and the indented portion of the second mold M2 contacts the other surface of the membrane 20.

In the case of the hot embossing process, either of the first mold M1 and the second mold M2 may be heated, or both the first mold M1 and the second mold M2 may be heated before combining. However, since the protruding shape of the first mold M1 has more influence on the formation of the microwell 21, it may be preferable to heat and use only the first mold M1. In addition, the temperature of the heated first mold M1 or second mold M2 may be preferably lower than the meltingpoint of the polymer nanofibers forming the membrane 20.

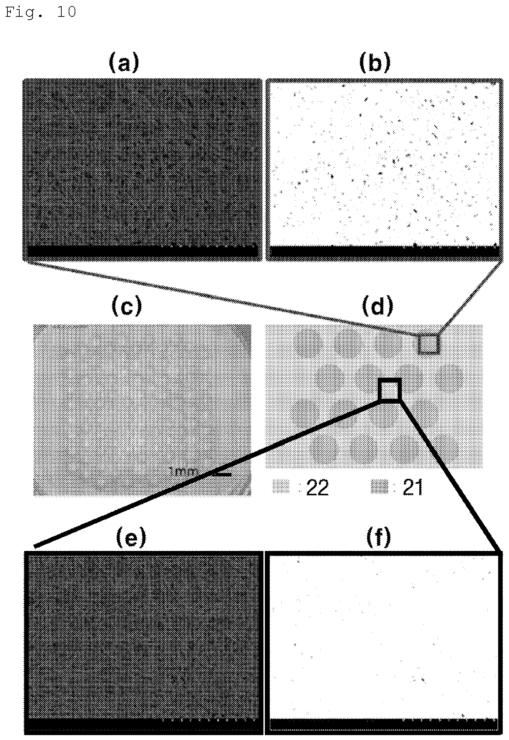

FIG. 10 shows images of a membrane 20 formed by the method for manufacturing a cell culture vessel according to an exemplary embodiment of the present invention. In this case, FIG. 10 (c) shows a plan image of the membrane 20, and FIG. 10(d) shows a plan view of the microwell 21 and the connection part 22, which are illustrated by enlarging FIG. 10 (c). In addition, FIG. 10(a) shows an enlarged image of the microwell 21, and FIG. 10(b) shows first pores formed in the microwell 21 of FIG. 10(a). In addition, FIG. 10(e) shows an enlarged image of the connection part 22, and FIG. 10(f) shows second pores formed in the connection part 22 of FIG. 10(e).

As illustrated in FIGS. 10(a) and 10(e), it can be confirmed that the membrane 20 formed by the method for manufacturing a cell culture vessel according to an exemplary embodiment of the present invention is formed in a network shape in which a plurality of polymer nanofibers are intertwined. In addition, referring to FIGS. 10(b) and 10(f), in the membrane 20 formed by the method for manufacturing a cell culture vessel according to an exemplary embodiment of the present invention, it can be confirmed that the number of first pores formed in the microwell 21 is greater than that of second pores formed in the connection part 22. In this case, it can be confirmed that the first porosity is increased by about 10 times or more compared to the second porosity.

The cell culture vessel and its manufacturing method were described with reference to FIGS. 1 to 10. For reference, in the examples referring to FIGS. 1 to 10 above, some mention was made of what conditions the porosity of parts constituting the membrane, such as microwells or connections, should have. However, the cell culture vessel according to the present invention only needs to have pores sufficient to allow fluid or material to pass through, and the porosity may have any value without any constraints. In other words, the presence of pores is naturally recognized in all examples mentioned in this detailed description, but it is understood that the difference in porosity is not an essential limitation in implementing a cell culture vessel.

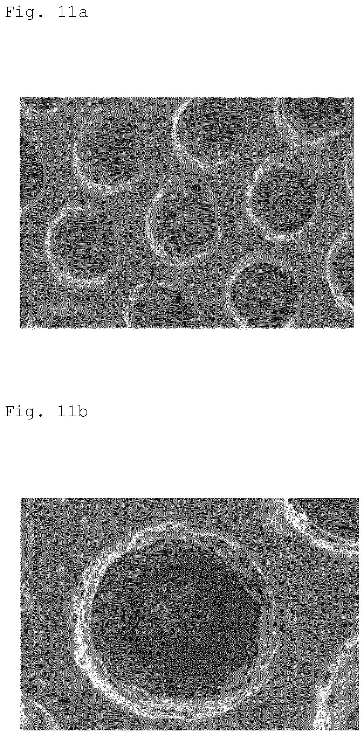

Hereinafter, another embodiment of a microwell and a plate will be described with reference to FIGS. 11 to 14.

In the description of FIG. 1, the porous microwell 21 and the plate 30 have been described above. In another embodiment, a cell culture vessel including a cell culture layer whose surface structure changes depending on temperature may be provided by coating the porous microwell 21 with a coating composition providing a surface structure that changes depending on temperature.

The cell culture layer having a surface structure that changes depending on temperature has, on the upper surface of the porous microwell 21, a surface structure which is suitable for cell culture at a temperature of 32°C to 40°C due to its high cell adhesion and is suitable for cell detachment and harvesting at a temperature of 0°C to lower than 32°C.

The cell culture layer may contain poly(N-isopropylacrylamide) (PNIPAAm), and exhibit time-dependent surface roughness changes, which correspond to a surface roughness of 4 to 37 nm at a temperature of 32°C to 40°C and a surface roughness of 4 um or more at a temperature of 0°C to lower than 32°C. Here, the surface roughness is a value measured by a non-contact atomic force microscope (Park Systems, Korea) using a PPP-NCHR cantilever at a frequency of 300 kHz or a BL-AC40TS cantilever at a frequency of 25 kHz.

The cell culture layer may contain a crosslinking agent in an amount of 1 to 5 wt%, preferably more than 1 wt% to less than 3 wt%, based on the total weight of the cell culture layer. If the content of the crosslinking agent is less than 1 wt%, a problem may arise in that cells do not adhere well to the cell culture layer, and if the content of the crosslinking agent is more than 5 wt%, a problem may arise in that detachment of cell spheroids does not occur well at a temperature below the lower critical solution temperature (LCST) .

Furthermore, the porous microwell 21 is permeable to a fluid, whereas portions other than the porous microwell 21 are not permeable to a fluid. Here, the pores of the porous microwell 21 may have an average pore size of 100 nm to 20 um, preferably 100 nm to 5 um. Due to such a pore size, the porous microwell can act as a selective permeable membrane that selectively permeates other materials without permeating single cells, and thus may serve as a material transfer barrier and channel.

In addition, when a fluid flows through the porous microwell 21 and the cell culture layer due to the difference in permeability between the porous microwell 21 and its surroundings as described above, a fluid concentration phenomenon may occur in the porous microwell. When this fluid concentration phenomenon occurs, oxygen and nutrients contained in the fluid may be smoothly supplied to cells proliferating and differentiating in the porous microwell, thereby further promoting cell proliferation and differentiation. In addition, a phenomenon also occurs in which cells are gathered in the porous microwells by this fluid concentrating phenomenon, thereby facilitating formation of cell aggregates or spheroids.

In addition, as shown in FIG. 1, the cell culture vessel of the present invention may comprise an upper chamber 100 and a lower chamber 200. The lower chamber 200 may be a chamber in which the upper chamber 100 is disposed or mounted so that a cell culture medium or the like may flow. Furthermore, according to the present invention, it is possible to a lower chamber in which a plurality of upper chambers may be disposed so that several types of cells may be cultured at the same time, and the number of the upper chambers is not limited as long as it is a number commonly used in the art.

Hereinafter, a method for manufacturing a three-dimensional cell culture vessel will be described. Specifically, the method may comprise steps of: providing an aqueous coating solution containing an N-isopropylacrylamide monomer, a crosslinking agent and the balance of water, wherein the content of the crosslinking agent is 1 to 5 parts by weight based on 100 parts by weight of a mixture of the N-isopropylacrylamide monomer and water; forming a coating layer on an upper surface of a porous microwell of a well plate chamber including the porous microwell at a bottom thereof using the aqueous coating solution; and forming a cell culture layer by irradiating the coating layer with UV light.

In this embodiment, it is possible to control the detachment rate of cells depending on the temperature change of the cell culture scaffold by adjusting the content of the crosslinking agent. As described above, the content of the crosslinking agent may be 1 part by weight to less than 5 parts by weight, preferably more than 1 parts by weight to less than 3 parts by weight, based on 100 parts by weight of a mixture of the N-isopropylacrylamide monomer and water. If the content of the crosslinking agent is less than 1 part by weight based on 100 parts by weight of the mixture of the N-isopropylacrylamide monomer and water, a problem may arise in that cells do not adhere well to the cell culture scaffold, and if the content of the crosslinking agent is more than 5 parts by weight, a problem may arise in that detachment of cell spheroids does not occur well at a temperature below the lower critical solution temperature (LCST).

Meanwhile, the crosslinking agent serves to polymerize the N-isopropylacrylamide monomer into polyisopropylacrylamide, and as the crosslinking agent, any crosslinking agent may be used without limitation as long as it may be used in a conventional method for production of polyisopropylacrylamide, such as homopolymerization, copolymerization, or cross-linked polymerization. Preferably, N,N-methylenebisacrylamide (MBAAm), tetramethylethylenediamine (TEMED), or a mixture thereof may be used as the crosslinking agent.

The aqueous coating solution may further contain a photoinitiator so that the monomer in the aqueous coating solution may be crosslinked by UV light. In this case, the content of the photoinitiator may be 0.01 to 0.1 parts by weight, preferably 0.01 to 0.05 parts by weight, based on 100 parts by weight of the mixture of the N-isopropylacrylamide monomer and water. If the content of the photoinitiator is less than 0.01 parts by weight, a problem may arise in that crosslinking by UV light does not occur, and if the content of the photoinitiator is more than 0.05 parts by weight, a problem may arise in that cells are killed by the toxicity of the crosslinking agent during subsequent cell culture.

Meanwhile, as the photoinitiator, any photoinitiator may be used without limitation as long as it may initiate crosslinking by UV light. For example, 2-hydroxy-1-1[4-(hydroxyethoxy)phenyl]-2-methyl-1-propanone may be used.

The step of forming the coating layer is not limited as long as the coating layer is formed by the aqueous coating solution so that a fluid passes through the coating layer into the porous microwell. For example, in the step of forming the coating layer, the coating layer may be formed using spin coating or bar coating. Furthermore, the cell culture layer formed by the coating layer is in the form of a hydrogel through which a solution migrates smoothly, and thus a fluid may flow through the porous microwell and the hydrogel.

**The** step of forming a cell culture layer by irradiating the coating layer with UV light is a step of polymerizing the monomer, and may be performed by UV irradiation so that an N-isopropylacrylamide polymer (polyisopropylacrylamide) may be formed. For example, the step may be performed by irradiating UV at 1,800 W for 10 minutes.

Meanwhile, for adhesion of cells and detachment of cultured cells, the 3D cell aggregate culture method may comprise a step of attaching and culturing 3D cell aggregates on the cell culture layer at a temperature of 32°C to 40°C, and detaching 3D cell aggregates from the cell culture layer at a temperature of 0°C to lower than 32°C, wherein the cells may be myoblasts, fetal fibroblasts, human umbilical vein endothelial cells, or human epidermal cells, without being limited thereto.

**<Example 1>**

In Example 1, an upper chamber including a porous microwell at the bottom was manufactured. Specifically, a porous microwell was manufactured by drilling polymethyl methacrylate (PMMA) to form a hole of a certain size and combining polymer nanofibers to the drilled PMMA by an adhesive.

Next, using the phase separation phenomenon that occurs in a solution containing a high concentration of an N-isopropylacrylamide monomer, an aqueous solution having a high content of N-isopropylacrylamide, separated from an aqueous solution having a low content of N-isopropylacrylamide, was prepared. The upper surface of the porous microwell of the upper chamber including the manufactured porous microwell was coated with the aqueous coating solution, thereby manufacturing a cell culture vessel in which a cell culture layer, to which cells adhere at a temperature of 32°C to 40°C and from which cells detach at a temperature of 0°C to lower than 32°C, was formed on the upper surface of the porous microwell.

Specifically, an N-isopropylacrylamide monomer and distilled water were mixed together at a mass ratio of 1:1, and stirred for 5 minutes so that the N-isopropylacrylamide monomer could be sufficiently dissolved in the distilled water. With the passage of time, the solution was stably separated into an aqueous solution containing a low concentration of N-isopropylacrylamide and an aqueous solution containing a high concentration of N-isopropylacrylamide. Finally, when the N-isopropylacrylamide aqueous solution was stably separated, the aqueous solution containing a high concentration of N-isopropylacrylamide was transferred into a vial using a pipette, thereby obtaining 5 ml of an aqueous N-isopropylacrylamide solution having a high content of N-isopropylacrylamide and having an N-isopropylacrylamide: water mass ratio of 87:13.

Next, 0.05 g of N,N'-methylenebisacrylamide (MBAAm) (1 part by weight based on 100 parts by weight of a mixture of the N-isopropylacrylamide monomer and water) as a crosslinking agent for making the monomer to react with UV light upon UV irradiation, and 0.005 g of 2-hydroxy-1-1[4-(hydroxyethoxy)phenyl]-2-methyl-1-propanone (0.01 part by weight based on 100 parts by weight of the mixture of the N-isopropylacrylamide monomer and water) as a photoinitiator, were added to the obtained aqueous N-isopropylacrylamide solution.

The composition prepared by adding the crosslinking agent and the photoinitiator to the aqueous N-isopropylacrylamide was applied thinly onto the polymer nanofibers using bar coating, and then irradiated with UV light for 10 minutes, thereby manufacturing a cell culture vessel in which a cell culture layer, to which cells adhere at a temperature of 32°C to 40°C and from which cells detach at a temperature of 0°C to lower than 32°C, was formed on the upper surface of the porous microwell.

FIGS. 11A and 11B show a cell culture vessel in which a cell culture layer, to which cells adhere at a temperature of 32°C to 40°C and from which cells detach at a temperature of 0°C to lower than 32°C, is formed on the upper surface of the manufactured porous microwell.

### <Experimental Example 1>

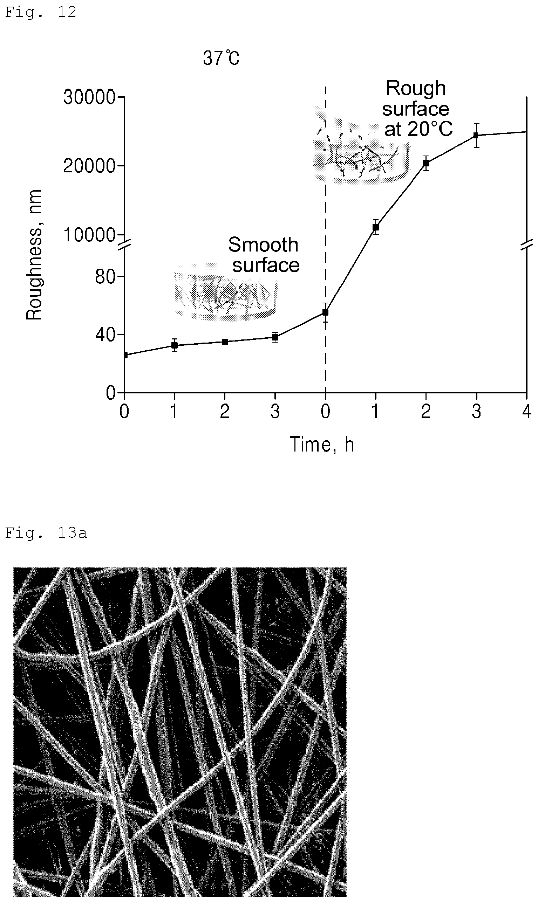

In order to measure changes in surface roughness, changes in the surface roughness of the cell culture layer of Example 1 depending on temperature and time were measured using an atomic force microscope (Park Systems, Korea), and the results are shown in FIG. 12.

As shown in FIG. 12, it could be confirmed that, at 37°C, which is a temperature exceeding the LSCT, there was little change in the surface roughness of the cell culture layer of Example 1, whereas, at 20°C, which is lower than the LSCT, there was an abrupt change in the surface roughness of the cell culture layer of Example 1.

### <Experimental Example 2>

The surface morphology of the cell culture layer in the cell culture vessel manufactured in Example 1 was compared in order to confirm the morphological difference of the cell culture layer, to which cells adhere at a temperature of 32°C to 40°C and from which cells detach at a temperature of 0°C to lower than 32°C.

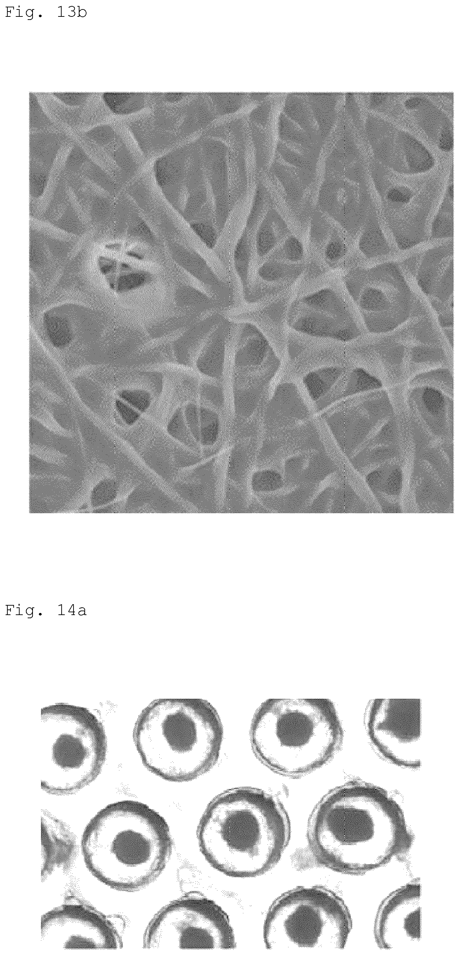

FIG. 13A shows polymer nanofibers before coating with the composition containing N-isopropylacrylamide, and FIG. 13B shows a cell culture layer formed by coating polymer nanofibers with the composition containing N-isopropylacrylamide. The cell culture layer is composed of a combination of polymer nanofibers and hydrogel.

### <Experimental Example 3>

The human liver cancer cell line (HepG2) was seeded in the cell culture vessel of Example 1 at 36°C. After 3 days, the resulting 3D cell aggregates were cultured. In order to harvest the cultured 3D human liver cancer cell aggregates, the upper chamber containing the 3D human liver cancer cell aggregates was moved to an environment having a temperature of 20°C.

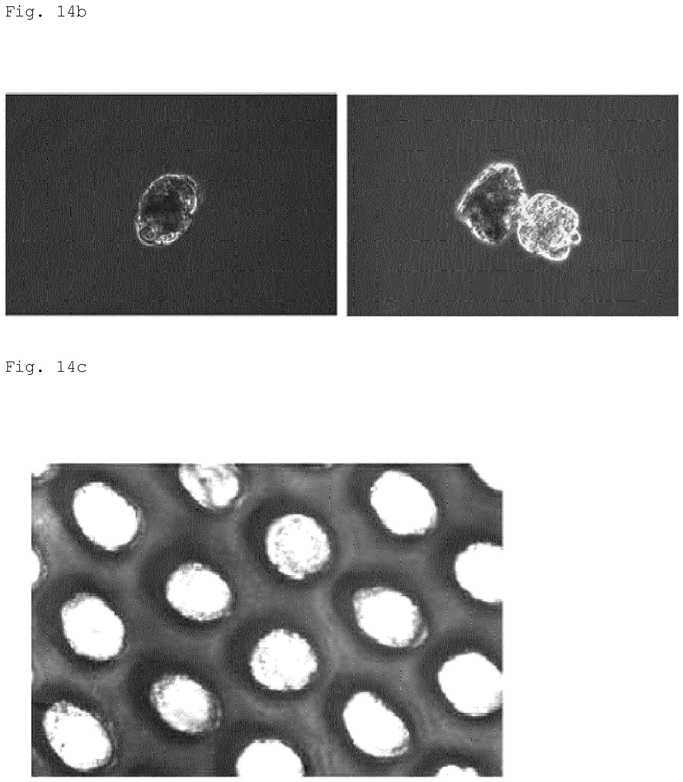

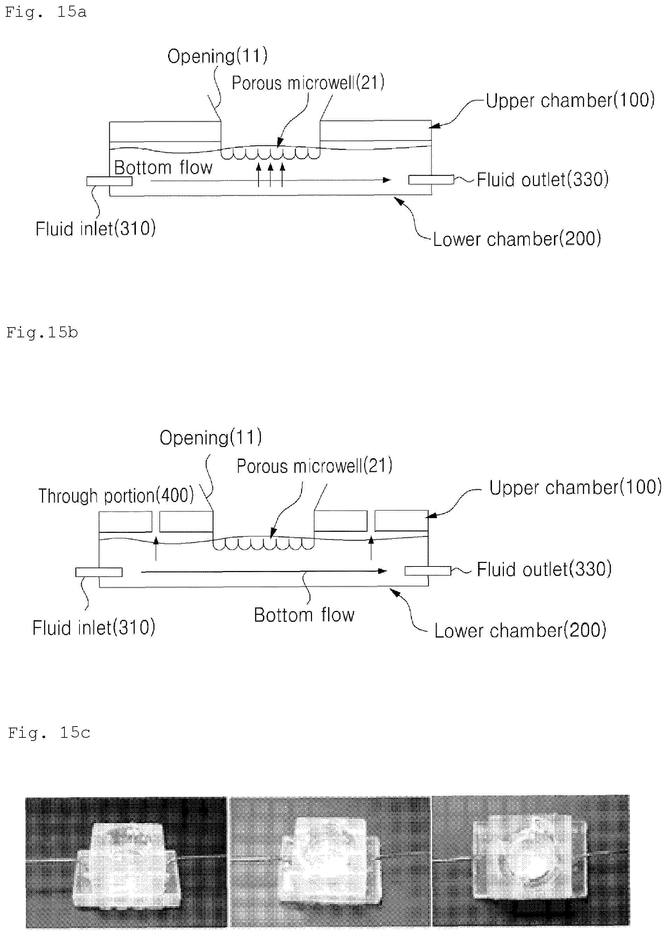

FIG. 14A shows an image of the cell culture vessel, taken during cell culture, and FIG. 14B shows an image of cultured cells. In addition, FIG. 14C shows an image of the cell culture vessel from which cultured cells have been detached. As shown in FIG. 15C, it could be confirmed that the cells cultured in the cell culture vessel of the present invention were easily detached.

Hereinafter, a cell culture device according to one embodiment of the present invention, more specifically, a cell culture device that improves the cell culture effect using a bottom flow, and a cell culture method using the same will be described with reference to FIGS. 15 to 18.

The present embodiment relates to a three-dimensional cell culture device capable of easily removing wastes generated during cell culture and smoothly supplying nutrients to the bottom of three-dimensional cell aggregates. Specifically, the present invention relates to a three-dimensional cell culture device comprising: an upper chamber 100 including an opening and a porous microwell; and a lower chamber 200 on which the upper chamber is disposed and in which a fluid may flow at the bottom of the porous microwell.

Referring to FIG. 15, the upper chamber 100 may have a structure in which a well-type chamber including an opening 11 and a porous microwell 21 is inserted through a plate. Furthermore, the bottom of the porous microwell 21 means a lower region of the porous microwell, which is a region within the lower chamber 200.

A through portion may be formed in the upper chamber 100 so that the fluid in the lower chamber 200 may contact the outside, and in this case, the through portion may be formed adjacent to the porous microwell 21 of the upper chamber 100 or may be formed at any position of the upper chamber 100. When the fluid is in contact with the outside as described above, the porous membrane may be prevented from being deformed by the physical force formed by the flow of the fluid. The position of the through-hole is not particularly limited as long as this prevention can be achieved.

The lower chamber 200 may further include a fluid inlet 310 and a fluid outlet 330, through which the fluid in the lower chamber 200 may flow. Furthermore, the fluid inlet 310 and the fluid outlet 330 may be connected to a device capable of causing a flow. For example, the lower chamber 200 may be connected to a device that induces fluid flow so that the fluid flows in the horizontal direction of the porous microwell 21. In this case, a pump or the like may be used as the device for inducing fluid flow, but any device may be used without limitation as long as it can cause the flow of the fluid.

As such, in the 3D cell culture device, the fluid in the lower chamber 200 may stably flow by the fluid flow induction device that allows the fluid to flow at the bottom of the lower chamber 200 to flow to the bottom of the porous microwell 21. Due to the permeability of the pores of the porous microwell 21, the fluid that flows as described above may discharge wastes, formed during 3D cell culture, from the upper surface of the porous membrane to the lower chamber 200, and may smoothly supply nutrients to the bottom of the 3D cell aggregates. Furthermore, the wastes may be discharged from the lower chamber 200 to the outside of the 3D cell culture device. For example, as the fluid flow induction device, a syringe pump, a peristaltic pump, or an agitator may be used.

In addition, in the 3D cell culture device, in order to disperse the pressure applied to the porous microwells during 3D cell culture and to efficiently remove formed wastes and supply nutrients formed during 3D cell culture, a through portion 400 may be formed in the upper chamber 100 so that the surface of the fluid in the chamber 200 may contact the outside. The through portion 400 provides a structure through which the surface of the fluid in the lower chamber 200 can come into contact with the outside. Due to the structure as described above, it is possible to prevent the porous microwell 21 from being deformed by the pressure applied to the upper chamber 100 during fluid flow, which adversely affects the settlement, proliferation and differentiation of cells during 3D cell culture.

Meanwhile, the present invention provides a three-dimensional cell culture method comprising a step of introducing cells and a cell culture medium into the porous microwell and culturing the cells, using the three-dimensional cell culture device of the present invention, wherein the cells may be myoblasts, fetal fibroblasts, human umbilical vein endothelial cells, human liver cancer cells (HepG2 cells), or human epidermal cells, without being limited thereto.

Hereinafter, the present invention will be described in more detail with reference to specific examples. The following examples are provided merely to assist in the understanding of the present invention, and the scope of the present invention is not limited thereto.

**<Example 2>**

In a three-dimensional cell culture device including an upper chamber 100 including a porous microwell 21 and a lower chamber 200 as shown in FIG. 15(a), HepG2 cells and DMEM (Dulbeco's Modified Eagle's Media, FBS 10%) were placed in the porous microwell 21, and then stabilized at 37°C for 48 hours to form three-dimensional spheroids. Thereafter, flow was applied to the lower chamber, followed by culture for 9 days.

**<Comparative Example 2>**

Three-dimensional HepG2 spheroids were cultured in the same manner as in Example 2, except that the bottom flow in Example 2 was not applied.

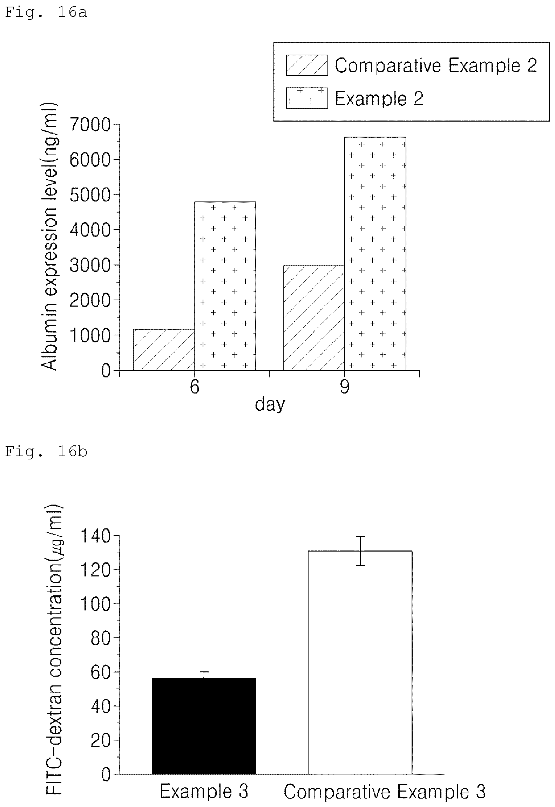

During culture in each of Example 2 and Comparative Example 2, the albumin expression level in the Hep G2 spheroids was measured on day 6 and day 9 during culture. The results of measuring the albumin expression level are shown in FIG. 16(a). As shown in FIG. 16(a), it could be confirmed that the albumin expression level in the Hep G2 spheroids cultured in Comparative Example 2 was 1,182.64 mg/ml on day 6 and 2,966.505 mg/ml on day 9, whereas the albumin expression level in the Hep G2 spheroids cultured in Example 2 was 4,813.99 mg/ml on day 6 and 6,644.55 mg/ml on day 9. That is, it could be seen that that the albumin expression level in the Hep G2 spheroids cultured in the three-dimensional cell culture device with a bottom flow was high, suggesting that the functionality of the three-dimensional Hep G2 spheroids was improved due to the bottom flow.

**<Example 3>**

In order to confirm that wastes can be efficiently removed from the three-dimensional cell culture device of the present invention by the bottom flow, 200 $\mu$g/ml of FITC-dextran (20 kDa) was placed in the porous microwell of the three-dimensional cell culture device shown in FIG. 15(a), and the bottom flow was performed for 3 hours, and then the concentration of FITC-dextran remaining in the porous microwell was measured.

**<Comparative Example 3>**

FITC-dextran was added in the same manner as in Example 3, except that the bottom flow in Example 3 was not applied. After 3 hours, the concentration of FITC-dextran remaining in the porous microwell was measured.

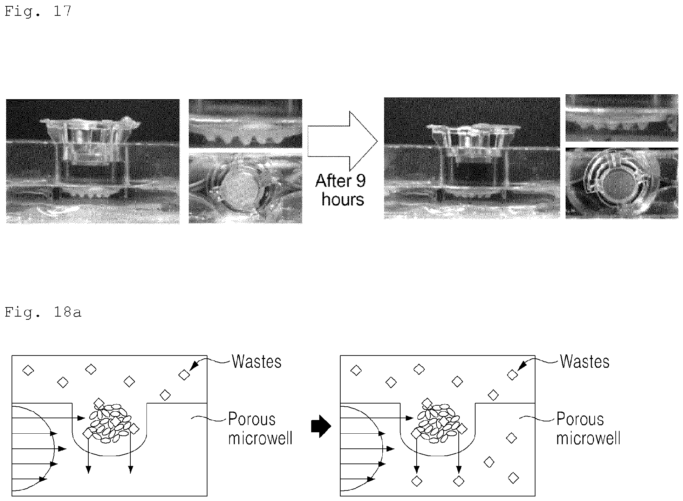

The results of visually examining the degree of waste accumulation in Example 3 are shown in FIG. 17, and the results of measuring the concentration of FITC-dextran in Example 3 and Comparative Example 3 are shown in FIG. 16 (b) . As shown in FIG. 16(b), it could be confirmed that, in the case of Example 4 with the bottom flow, the concentration of FITC-dextran remaining in the porous microwell was 55.6199±3.3429 mg/ml, whereas, in the case of Comparative Example 3, the concentration of FITC-dextran remaining in the porous microwell was 131.435±7.80245 mg/ml.

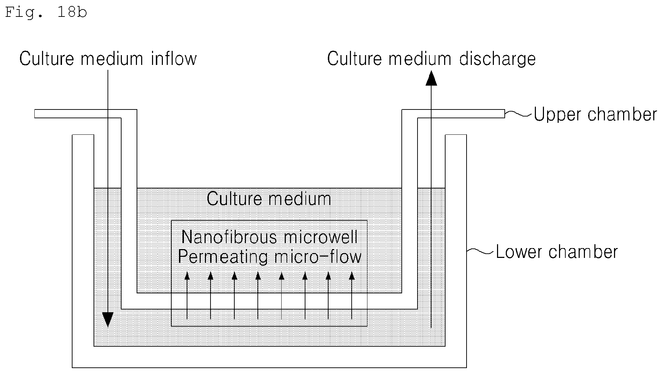

That is, it could be confirmed that, when there was bottom flow, the concentration of FITC-dextran remaining in the porous microwell was significantly lower than that in the case in which there was no bottom flow. This suggests that, even with equal amounts of culture medium, cell wastes in the porous microwell could be efficiently removed when there was bottom flow. As shown in FIG. 18(a), it could be confirmed that wastes on the upper surface of the porous microwell were removed to the lower chamber by the fluid flow induction device and the porous microwell. FIG. 18(a) shows a state in which wastes present in the porous microwell are removed through the pores when the culture medium flows at the bottom, that is, a state in which wastes pass through the pores along the flow of the fluid, and thus are removed from the inside of the microwell. Meanwhile, FIG. 18(b) shows a phenomenon in which a culture medium naturally diffuses into the microwell when the culture medium flows as the bottom flow in the lower chamber, and shows that, when there is the bottom flow of the culture medium, wastes may be removed through the pores of the membrane and diffusion of the culture medium may also occur at the same time. Referring to FIGS. 18(a) and 18(b), it can be seen that the direction in which wastes pass through the pores and the direction in which the culture medium passes through the pores are opposite to each other.

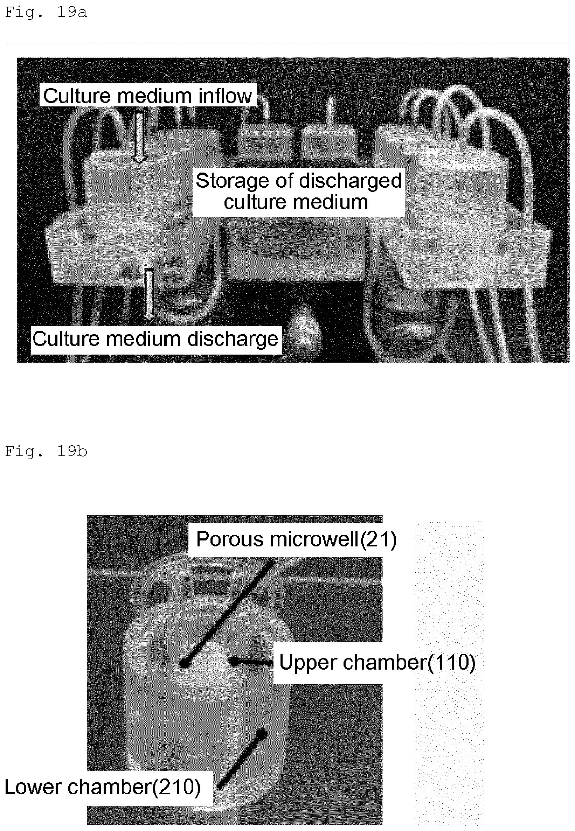

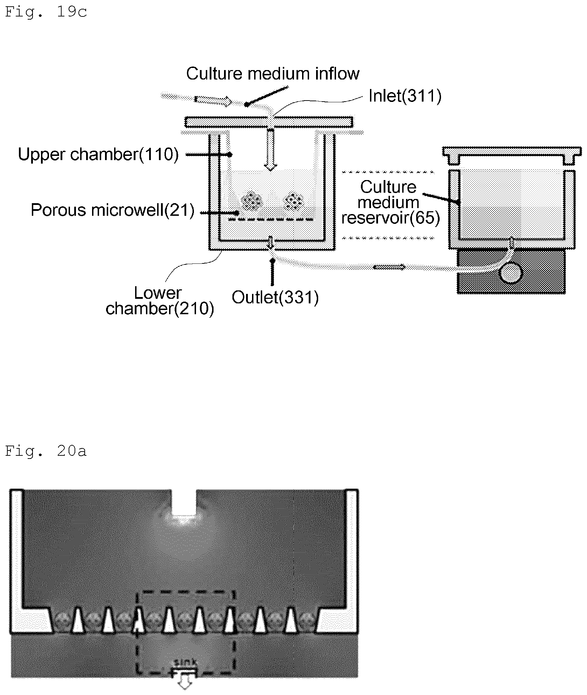

Hereinafter, a cell culture device and cell culture method according to another embodiment of the present invention will be described with reference to FIGS. 19 to 29.

According to this embodiment, it is possible to solve the problem that the loss of cells and cell aggregates occurs due to a culture medium flow caused by pipetting when using a conventional cell culture medium replacement method of removing the used cell culture medium from the upper surface of the microwell by pipetting and injecting a fresh cell culture medium during cell culture. In addition, it is possible to provide uniform cellular microenvironments such as nutrients by the flow around cell aggregates, in which the cellular microenvironments are continuously induced by passage through the porous microwell. Therefore, according to the present invention, it is possible to minimize the loss of cells as described above during cell culture, and at the same time, continuously induce uniform microenvironments around cell aggregates, thereby culturing 3D cell aggregates showing phenomena more similar to *in vivo* phenomena than 2D culture.

Referring to FIG. 19, the cell culture device according to this embodiment comprises an upper chamber 110 including an opening, a porous membrane, and a porous microwell 21 having a cell culture space that accommodates a culture medium. In addition, the cell culture device comprises a lower chamber 210 having a space in which the upper chamber 110 is disposed. The cell culture device is configured such that a fluid introduced into the upper chamber 110 permeates the porous microwell 21 in the upper chamber 110 and flows into the lower chamber 210, and the fluid in the lower chamber 210 is discharged.

In addition, the present invention provides a method of culturing 3D cell aggregates using the cell culture device of FIG. 19, the method comprising steps of: seeding cells on the porous membrane of the upper chamber 110; introducing a cell culture medium into the upper portion of the upper chamber 110 through an inlet 311 or an opening; and discharging the cell culture medium, that permeated the porous microwell in the upper chamber 110 and flowed into the lower chamber 210, from the lower chamber 210. In addition, in the method of culturing 3D cell aggregates, the cell culture medium flows by the cell culture medium that is discharged from the lower chamber 210. Furthermore, the method may further comprise a step of introducing the cell culture medium into the lower chamber 210.

The 3D cell culture device according to one embodiment of the present invention is configured such that the fluid introduced into the upper portion of the upper chamber 110 permeates the porous microwell 21 in the upper chamber and flows into the lower chamber 210, and furthermore, the fluid in the lower chamber 210 is discharged. In this case, the fluid in the lower chamber 210 may be discharged through a fluid outlet provided in the lower chamber 210 or may be discharged through a gap region between the upper chamber 110 and the lower chamber 210. For example, it may be discharged through an upper portion of the gap region.

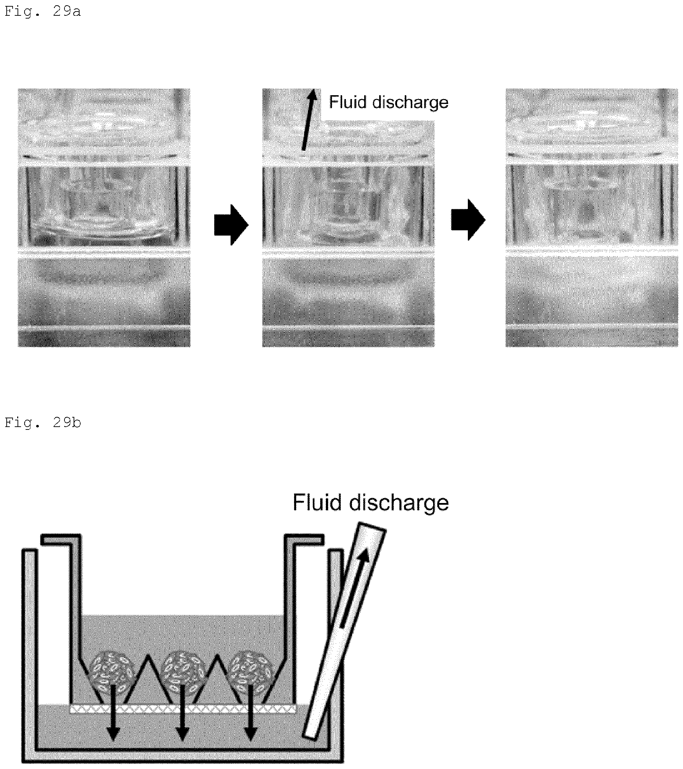

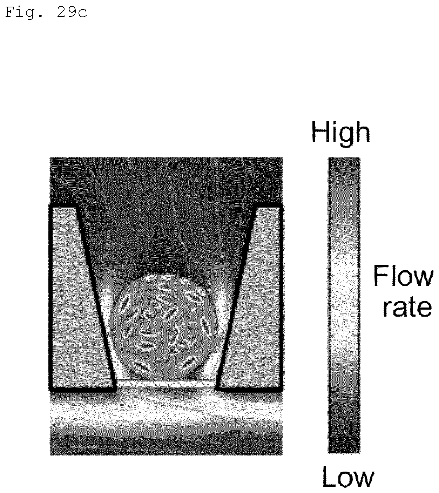

The fluid outlet may be formed at any location of the lower chamber 210. For example, it may be provided at the bottom of the lower chamber 210 as shown in FIG. 19(c), without being limited thereto. Meanwhile, when this fluid outlet is not provided in the lower chamber 210, as shown in FIGS. 29(a) to 29(c), the fluid may be discharged through the gap region between the upper chamber 110 and the lower chamber 210 to form a fluid flow. In this case, the size of each chamber may be controlled such that a gap exists between the sidewall of the upper chamber 110 and the sidewall of the lower chamber 210 and the fluid may be discharged from the top of the air-contacting interface in the gap region between them.

In summary, the 3D cell culture device according to the present invention may have a discharge structure configured to discharge a fluid from the lower chamber 210. In this case, the discharge structure may be a structure configured to allow a fluid to flow through the fluid outlet into the lower chamber 210, or a structure configured to allow a fluid to flow by discharging the fluid through a gap region between the upper chamber 110 and the lower chamber 210.

Meanwhile, there may be other discharge structures that are not specifically mentioned in the detailed description of the invention, and detailed conditions for the discharge structure are not limited as long as the flow of the fluid containing a cell culture medium can maintain the downward flow from the upper chamber 110 to the lower chamber 210.

For reference, in this embodiment, the porous membrane may be composed of a nanofiber network which is a porous membrane having a porosity of 20% to 60%, for example, 30% to 50%. If the porosity is less than 20%, a problem may arise in that the hydraulic conductivity is lowered, and thus the pressure applied to the porous membrane increases, resulting in a decrease in the viability of cell aggregates being cultured on the porous membrane.

In addition, the porous membrane may have an average pore size of 10 nm to 10 um. That is, as the porous membrane has an average pore size within the above range, it may act as a selective permeable membrane that selectively permeates other materials such as nutrients and growth factors in a cell culture medium without permeating single cells. As a result, the porous membrane enables the formation of three-dimensional aggregates by inducing aggregation of single cells thereon, and may serve as a material transfer barrier and channel after formation of the aggregates.

The porous membrane may have a high hydraulic conductivity within the above-described porosity and average pore size ranges of the present invention. In addition, the porous membrane may have a hydraulic conductivity of 1 to 20 um s$^{-1}$. When the hydraulic conductivity is less than 1 um s$^{-1}$, high pressure may be caused in the porous membrane, and if a pressure of 1,000 Pa or more is applied to the porous membrane and cell aggregates being cultured, adverse effects such as lowering the viability of cells may occur.

The porous membrane may be a nanofiber network for cell culture, and a method for manufacturing the same is not particularly limited, but the porous membrane may be composed of, for example, polymer nanofibers formed by electrospinning.

The method of manufacturing the nanofiber network may comprise a step of electrospinning, for example, a solution obtained by dissolving polycaprolactone in a mixed solvent of chloroform and methanol (3/1 vol/vol) to a concentration of 4 to 10 wt%. The electrospinning is preferably performed at a polymer solution discharge rate of 0.1 to 2.0 ml hr$^{-1}$ under a voltage of 10 to 30 kV. If the voltage is lower than the lower limit of the above range, a problem may arise in that it is difficult to manufacture uniform nanofibers, and if the voltage is higher than the upper limit of the above range, a problem may arise in that it is difficult to manufacture stable nanofibers, due to non-uniform stacking of nanofibers.

In the porous microwell, all or part of a concave portion may be composed of a porous membrane. Preferably, when the porous microwell, that is, the opening of the upper chamber, is disposed to face upward, the surface forming the bottom is formed of a porous membrane.

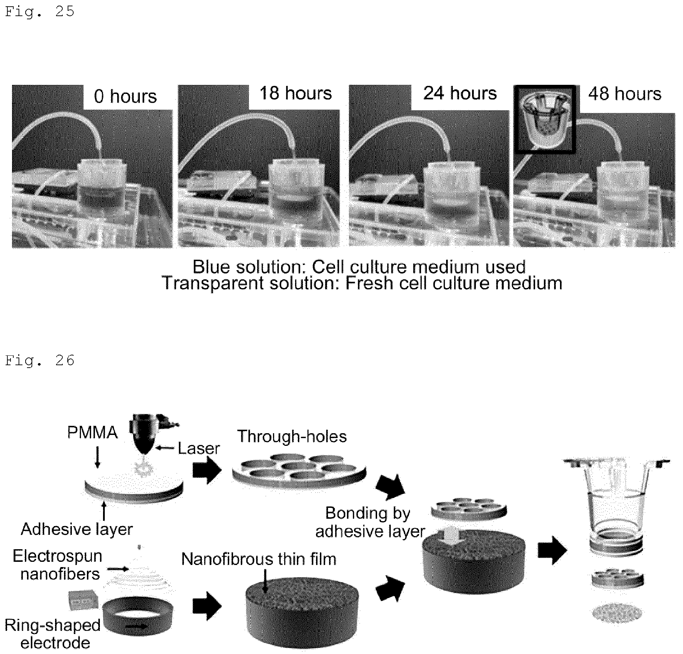

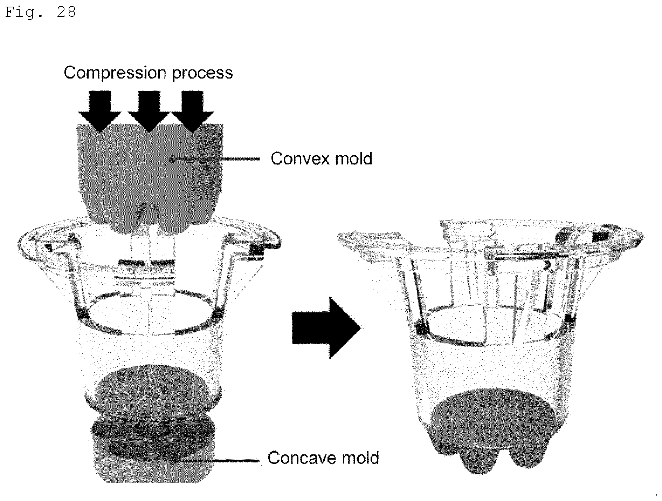

Meanwhile, the porous membrane constituting the bottom surface of the upper chamber 110 of the present invention may be formed to have a protrusion and/or a concave portion. For example, as shown in FIG. 28, a concave portion may be formed using a porous membrane, or as shown in FIG. 26, a concave portion may be formed by adding a side wall or a protrusion, which is capable of forming a compartment corresponding to the concave portion, onto a porous membrane. In this case, the material of the side wall or the protrusion may be porous or non-porous and is not particularly limited. Preferably, the bottom surface of the concave portion may be composed of a porous membrane, and the side wall or protrusion may be porous or non-porous.

Preferably, the bottom surface of the concave portion may be composed of a porous membrane, and the side wall or protrusion may be porous or non-porous. For example, FIGS. 21(a) and 21(b) illustrate an example of a case in which the sidewall of a concave portion formed by stacking an additional layer having through holes is not composed of a porous material, and FIG. 21(c) illustrates a case in which a concave portion is formed in a porous membrane itself and the concave portion and portions other than the concave portion are all composed of a porous material.

Porous microwells may be manufactured by, for example, combining a porous membrane, fabricated by electrospinning, with an array of through-holes, as shown in FIG. 26, without being limited thereto. Meanwhile, as shown in FIG. 21(c), formation of the concave portion and protruding portion composed of a porous material may be performed, for example, by a compression process using a mold, as shown in FIG. 28.

The upper surface of the porous membrane of the porous microwell is a region that functions as a cell culture layer. When the porous membrane has a protrusion and a concave portion as described above, cells may be more easily seated in the formed concave portion and may be cultured after 3D cell aggregates are formed by inducing aggregation of single cells in the porous microwell. A more preferred porous membrane of the present invention has a protrusion and a concave portion, and all the surfaces thereof are composed of a porous membrane.

Meanwhile, the lower chamber 210 has a space in which the upper chamber 110 is disposed, and when a fluid outlet is provided in the lower chamber 210, a culture medium may flow while a culture medium-containing fluid in the lower chamber 210 having the upper chamber 110 disposed therein is discharged to the outside through the fluid outlet 331. To this end, a device capable of inducing flow may be additionally connected to the upper chamber and/or lower chamber 210. For example, a device that inject a cell culture medium into the upper chamber 110 at a constant flow rate may be added, and the lower chamber 210 may be connected to a fluid flow control device that allows a fluid introduced into the upper chamber 110 to permeate the porous membrane of the upper chamber 110 and to be discharged at a constant flow rate while flowing into the lower chamber 210. The fluid may be discharged into, for example, a culture medium reservoir. In this case, any device capable of inducing the flow of the fluid may be used without limitation. For example, the fluid flow induction device may be a syringe pump, a peristaltic pump or an agitator.

The three-dimensional cell culture device according to the present invention may further comprise a culture medium reservoir 65 disposed on the same plane as the lower chamber 210 and being in fluid communication with the lower chamber 210, wherein the height of the culture medium in the culture medium reservoir 65 may be set to correspond to the level of the culture medium in the lower chamber 210.

The level of the culture solution in the lower chamber 210 may correspond to a height of 1 to 20 mm, for example, 1 to 19 nm, preferably 1 to 18 mm, for example, 1 to 8 mm, in the upward direction from the porous membrane at the bottom of the upper chamber 110. If the level of the culture medium is lower than the lower limit of the above preferred range, it may be impossible to smoothly supply nutrients to cell aggregates during culture, and if the level is higher than the upper limit of the above preferred range, overflow and overuse of the culture medium may occur.

In addition, the spacing between the porous membrane at the bottom of the upper chamber 110 and the bottom of the lower chamber 210 may be 0.1 to 8 mm, for example, 0.2 to 7 mm, preferably 1 to 5 mm. If the spacing is smaller than the lower limit of the above preferred range, the flow of the cell culture medium may be limited and discharge of the cell culture through the outlet may be impossible. In addition, if the spacing is larger than the upper limit of the above preferred range, overuse of the culture medium may occur.

In this case, the fluid, that is, the cell culture medium, may flow while maintaining a constant rate. To this end, the inflow and outflow of the fluid may be controlled at a constant rate. For example, the fluid may flow at a rate of 0.0001 to 1 ml hr$^{-1}$, for example, 0.001 to 1 ml hr$^{-1}$, preferably 0.01 to 1 ml hr$^{-1}$. If the flow rate of the fluid is less than 0.0001 ml hr$^{-1}$, a problem may arise in that the cell culture medium is not supplied in an amount required for the cell aggregates, and if the flow rate is more than 1 ml hr$^{-1}$, an excessive shear stress caused by the flow of cell aggregates may adversely affect cell aggregates. A shear stress that does not adversely affect cells, for example, a shear stress of 0.001 to 10 dyne cm$^{-2}$ may positively affect cell differentiation and proliferation.

Meanwhile, the cell culture medium is preferably applied to the porous membrane at a pressure of less than 1,000 Pa, and the smaller the pressure, the more preferable. For example, the pressure may be 1 Pa to 100 Pa. If the pressure is higher than 1,000 Pa, a problem may arise in that cell viability is lowered.

In the three-dimensional cell aggregate culture device and the method of culturing three-dimensional cell aggregates using the device according to the present invention, cell lines, for example, myoblasts, fetal fibroblasts, and umbilical vein endothelial cells vein endothelial cells, liver cancer cells (HepG2 cells), epidermal cells, or a mixture of at least two of these cell types may be used. In addition, not only cell lines but also primary cells such as liver, pancreas, or small intestine cells may be used. Finally, stem cells, such as induced pluripotent stem cells, mesenchymal stem cells, a mixture of at least two of these cell types may be cultured, without being limited thereto, and spheroids or organoids, which are three-dimensional cell aggregates, may also be cultured.

Meanwhile, the method of culturing 3-dimensional cell aggregates using the three-dimensional cell aggregate culture device according to this embodiment may comprise steps of: seeding cells on the porous membrane of the upper chamber 110; and introducing a cell culture medium into the upper portion of the upper chamber 110 through the inlet 311 or the opening.

The cells may be seeded and cultured on the porous membrane, and the seeding may be performed by seeding the cells to be cultured onto the porous membrane prior to the step of introducing the fluid into the upper chamber 110. Thus, the cells may be applied to the above-described 3-dimensional cell culture device, and the method of seeding the cells may be performed by a method known used in the art. For example, the seeding may be performed using a micropipette.

The cells seeded by the above method form three-dimensional cell aggregates within several hours to several days. After formation of cell aggregates, the flow of the cell culture medium by the device of the present invention may be applied.

Hereinafter, the present invention will be described in more detail through specific examples. The following examples are only to assist in understanding of the present invention, and the scope of the present invention is not limited thereto.

**EXAMPLES**

**1. Fabrication of porous membrane**

Polycaprolactone (PCL; Mn = 80,000 g mol$^{-1}$), chloroform, and methanol were purchased from Sigma-Aldrich (USA). A PCL solution for electrospinning was prepared by dissolving PCL in a mixture of chloroform/methanol (3/1 vol/vol) at a concentration of 7.5 wt%. The prepared PCL solution was put into a 5-ml gastight syringe (Hamilton) and discharged using a commercial electrospinning machine (ES-robot, NanoNC) at a flow rate of 1 ml h$^{-1}$ through a 23-gauge metal needle placed at a distance of 10 cm from a ring-shaped electrode having a diameter of 5 cm. Electrospinning was performed by applying a high voltage of 15 kV between the metal needle and the ring-shaped electrode using the commercial electrospinning machine. As-electrospun PCL nanofibers were deposited in the ring-shaped electrode to produce a gas- and mass-permeable nanofibrous membrane. Electrospinning was performed at a relative humidity of 50 to 60% and a temperature of 20 to 25°C. FIG. 22 shows micrographs of nanofiber networks fabricated at different spinning times. As shown therein, it was confirmed that the nanofiber diameter was about 800 to 1,000 nm, and as the spinning time increased, the average pore size and porosity decreased, whereas the average thickness increased. FIG. 22 shows the structures (FIG. 22(a)), porosities (FIG. 22(b)), hydraulic conductivities (FIG. 22(c)) and induced pressures (FIG. 22(d)) of the nanofibrous networks fabricated at different electrospinning times. Here, the porosity was determined by converting the enlarged microscope image into a binary image, and then calculating the area fraction of pores, generated by the nanofiber network, relative to the area of the nanofiber network by using Image J software (NIH, USA).

## 2. Manufacturing of upper chamber including porous microwell

As shown in FIG. 26, a series of processes were performed to manufacture an upper chamber including a porous membrane having a cell culture space for accommodating the porous membrane obtained in 1 above and a culture medium. Specifically, to fabricate a porous membrane region at the bottom of an upper chamber, an adhesive was applied to a 500-$\mu$m-thick polymethyl methacrylate (PMMA) plate (Acryl Choika, South Korea), and the adhesive-applied PMMA plate was drilled by a laser cutter (ML-7050A, Machineshop, South Korea) to form an array of through-holes. The through-hole array and the porous membrane were combined together using the applied adhesive, thereby forming concave portions having a bottom surface composed of a porous membrane as shown in FIG. 21.

A series of processes of fabricating another type of porous membrane region at the bottom of an upper chamber are shown in FIG. 28. More specifically, the flat porous membrane obtained in 1 above was subjected to a compression process using a convex mold and a concave mold, thereby fabricate an upper chamber bottom comprising a porous membrane in which convex and concave portions, whose all surfaces are composed of the porous membrane, were formed. In this case, the concave mold was fabricated by drilling a 10-mm-thick polymethyl methacrylate (PMMA) plate (Acryl Choika, South Korea) by a machining device (EGX-350, Roland, USA), and the convex mold composed of polydimethylsiloxane (PDMS) was fabricated by pouring a mixture of PDMS and a curing agent (10:1 w/w) (Sylgard 184, Dow Corning, USA) into a concave mold and curing the mixture at 55°C for 12 hours. The remaining side portion having an opening for an upper chamber was manufactured using an injection molding machine (SE50D, Sumitomo, Japan). The obtained side portion and the upper chamber bottom obtained as described above were combined together using an adhesive to obtain an upper chamber including a porous microwell.

## 3. Manufacturing of 3-dimensional cell aggregate culture device

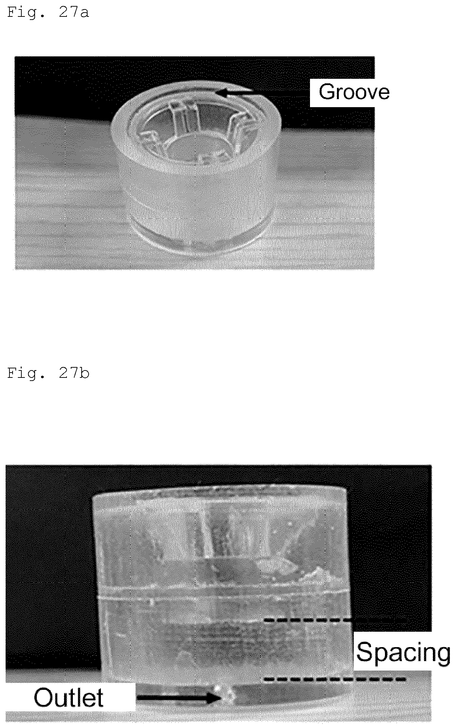

To fabricate a lower chamber composed of polydimethylsiloxane (PDMS) and a cover thereof, a mixture of PDMS and a curing agent (10:1 w/w) (Sylgard 184, Dow Corning, USA) was poured into a mold and cured at 55°C for 12 hours. A mold for a lower chamber and a cover was fabricated from a 20-mm-thick PMMA plate using a machining device (EGX-350, Roland, USA). The lower chamber was fabricated to have a size of 30 mm × 30 mm × 30 mm. In this case, a groove with a depth of 2 mm was designed on the upper surface (FIG. 27(a)), and the spacing between the bottom surface of the lower chamber and the porous membrane of the porous microwell fabricated in 2 above was set to 8 mm (FIG. 27(b)). An outlet having a size of 1 mm was formed by drilling the center of each of the lower chamber bottom and the cover using a biopsy punch (Miltex, USA). A tube (Biokonvision, South Korea) was connected through the hole of the cover, and a syringe pump (KDS200, KD Scientific, USA) injected a cell culture medium at a flow rate of 0.062 ml h$^{-1}$ through the tube.

The hole in the bottom of the lower chamber was also connected to the tube so that the cell culture medium could be transferred to a culture medium reservoir. In addition, a Z-stage (Sciencetown, South Korea) was provided beneath the culture medium reservoir, and the level of the culture medium in the reservoir was set to correspond to the level of the culture medium in the lower chamber, so that the level of the culture medium in the lower chamber could be kept constant even though the culture medium was continuously introduced from the syringe pump during culture.

## 4. Cell culture using aggregate culture device

<Example 4>

A three-dimensional cell culture device comprising a culture medium reservoir capable of storing a discharged culture medium, in addition to the upper chamber including the porous microwell prepared in 1 above and the lower chamber, was prepared as shown in FIG. 19. In this case, the height of the culture medium reservoir was set so that the level of the lower chamber from the bottom surface of the lower chamber corresponded to 12 mm. Regarding the cell culture device obtained as described above, the terms "cell culture device of the present invention" and "cell culture device of Example 4" will be used interchangeably.

In the three-dimensional cell culture device, liver cells (HepG2) and DMEM (Dulbeco's Modified Eagle's Media, FBS 10%) as a cell culture medium were introduced into the porous microwells and then maintained in a static environment at 37°C for 48 hours without causing flow, thereby forming three-dimensional HepG2 aggregates, that is, HepG2 spheroids. Thereafter, the cell culture medium was continuously injected into the upper chamber at a constant flow rate of 0.062 ml hr$^{-1}$ by a syringe pump. Next, the HepG2 spheroids were cultured for 8 days while discharging the fluid to the culture medium reservoir through the lower chamber having the fluid outlet 331 formed therein in the downward direction in order to discharge the fluid in an amount corresponding to the injected amount.

**<Comparative Example 4>**

HepG2 spheroids were cultured in the same manner as in Example 4, except that an impermeable microwell composed of the bottom surface of an impermeable PMMA plate rather than a porous membrane was used, and cell culture medium replacement was performed by pipetting without using devices for inducing the flow of the cell culture medium, such as a syringe pump or a culture medium reservoir.

**Experimental Example**

(1) Confirmation of fluid exchange within three-dimensional cell culture device over time

In the three-dimensional cell culture device of Example 4, an experiment was conducted to confirm the flow change (fluid exchange confirmation) in the three-dimensional cell culture device when a fresh fluid was injected into the upper chamber at a flow rate of 0.062 ml hr$^{-1}$.

As shown in FIG. 25, it could be confirmed that the blue solution existing in the chamber containing the cell culture aggregates was gradually exchanged with the newly injected transparent solution. (In the black and white drawing, it can be seen that the dark colored solution gradually changed to a bright color as time passed from the left to the right of FIG. 25). In addition, it could be confirmed that the level of the solution in the lower chamber was kept constant during this process.

**(2) Analysis of flow of cell culture medium in Example 4**

In order to apply a computational fluid analysis method for measuring the flow of the cell culture medium in the three-dimensional cell culture device of the present invention, details corresponding to the surface velocity (0 m s$^{-1}$ to 8.11 e$^{-6}$ m s$^{-1}$), flow direction (black cone shape), and streamline (white line) in the three-dimensional cell culture device of Example 4 were analyzed and calculated through COMSOL Multiphysics software (Version 5.0, USA).

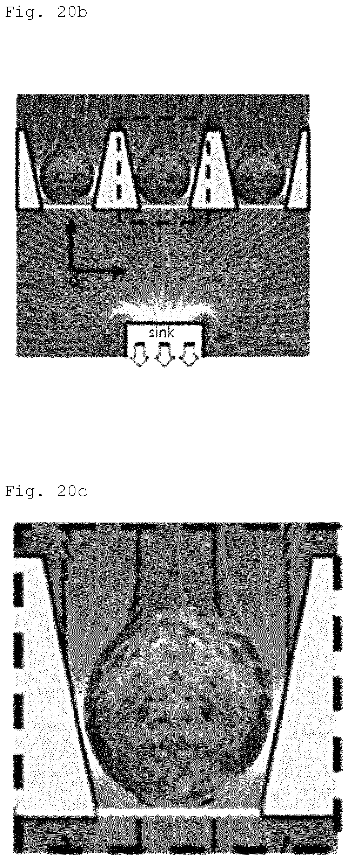

As a result, as shown in FIG. 20, it could be confirmed that, in the three-dimensional cell culture device of the present invention, the cell culture medium smoothly flowed from the upper chamber to the lower chamber.

**(3) Comparison of degree of cell loss during cell culture**

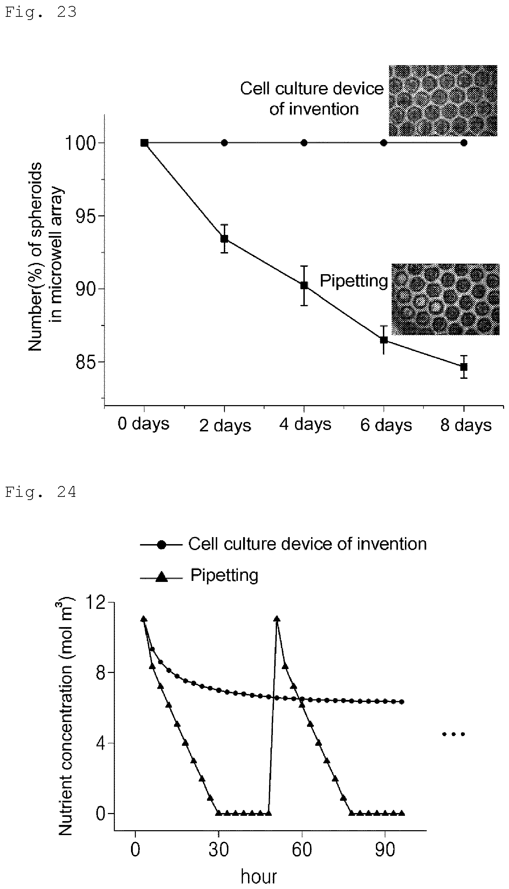

The degrees of loss of liver cell spheroids in Example 4 and Comparative Example 4 were compared every 2 days, and the results are shown in FIG. 23.

As shown in Figure 23, it could be confirmed that, in Example 4, there was no loss of liver cell spheroids, but in Comparative Example 4, spheroids were continuously lost, and a loss of about 15% of HepG2 cell spheroids on day 8 occurred.

**(4) Comparison of time-dependent nutrient concentration around three-dimensional cell aggregates**

In Example 4 and Comparative Example 4, COMSOL Multiphysics software (Version 5.0, USA) was used to measure and comparatively analyze the time-dependent nutrient (glucose) concentration around HepG2 spheroids. The values used in this process are shown in Table 1 below.

[Table 1]

| Molecules | MW [kDA] | Diffusion coefficient [$\mu m^2 s^{-1}$] | Initial concentration [mol m$^{-3}$] in culture medium | Consumption rate [mol m$^{-3}$s$^{-1}$] |
|---|---|---|---|---|
| Glucose | 0.18 | 580 | 11.1 | 5.2E-3 |

As shown in FIG. 24, it was numerically predicted that, in the cell culture device of Example 4, a uniform nutrient concentration was induced around the HepG2 cell aggregates after 36 hours, but in Comparative Example 4, the change in nutrient concentration before and after the pipetting period was severe, and thus non-uniform nutrient concentrations were induced around cell aggregates.

**(5) Measurement of hydraulic conductivity and induced pressure of porous membrane in three-dimensional cell culture device of Example 4**

An experiment was conducted to check the pressure applied to the membrane in the three-dimensional cell culture device when a fresh fluid was injected into the upper chamber at a flow rate of 0.062 ml hr$^{-1}$. To this end, the hydraulic conductivity was measured outside the device of the present invention using the Falling-head method as follows.

Specifically, the time until water having an initial pressure head ($h_i$) permeates a porous membrane and reaches a final pressure head ($h_f$) was measured, and the hydraulic conductivity was calculated using the following Equation based on Darcy's law.

$$K = \frac{L}{\Delta t} \times \ln \frac{h_f}{h_i}$$

(Equation 1)

wherein K is the hydraulic conductivity of the porous membrane, L is the thickness of the porous membrane, and t is the time from $h_i$ to $h_f$. In this experiment, $h_i$ and $h_f$ of 100 mm and 10 mm, respectively, were used. Based on the calculated hydraulic conductivity, the permeability of the porous membrane was calculated using the following Equation 2.

$$k = \frac{K\mu}{\rho g}$$

(Equation 2)

wherein k is the permeability of the porous membrane, $\mu$ is the viscosity of the cell culture medium, $\rho$ is the density of the cell culture medium, and g is gravitational acceleration.

Based on the cell culture medium permeating the porous membrane at a flow rate of 0.062 ml hr$^{-1}$, the measured hydraulic conductivity and the calculated permeability, the pressure applied to the porous membrane was calculated using the following Equation 3 (Kozeny-Carman equation).

$$v = \frac{k\Delta p}{\mu L}$$

wherein k is the flow rate of the fluid permeating the porous membrane, $\mu$ is the viscosity of the cell culture medium, L is the thickness of the porous membrane, and p is the pressure applied to the porous membrane.

As shown in FIG. 22, it was confirmed that the pressure applied to the porous membrane, that is, the hydraulic repulsive force, decreased as the network of nanofibers constituting the porous membrane became coarser, that is, as the electrospinning time was shorter, that is, the porosity and the hydraulic coefficient were higher. As a result of analyzing these values comparatively with those of a commercial porous membrane which is not the porous membrane of the present invention, it could be confirmed that a much lower hydraulic repulsive force was applied to the porous membrane of the present invention, and that the porous membrane composed of a nanofiber network was suitable for the device of the present invention.

**Claims**

1. A 3D cell culture device, comprising:

   an upper chamber, comprising porous microwell comprising an opening, a porous membrane, and cell culture space for accommodating a culture medium;
   a lower chamber, comprising a space in which the upper chamber is disposed;
   wherein fluid flowing into the upper part of the upper chamber passes through the porous microwell in the upper chamber and flows into the lower chamber, and
   wherein the fluid in the lower chamber is discharged and the fluid flows.

2. The 3D cell culture device according to claim 1, wherein the fluid in the lower chamber is discharged through a fluid outlet provided in the lower chamber, or is discharged through a gap region between the upper chamber and the lower chamber.

3. The 3D cell culture device according to claim 2, further comprises a culture medium reservoir disposed on the same plane as the lower chamber, and in fluid communication with the fluid outlet of the lower chamber, and
   wherein the level of the culture medium in the culture medium reservoir is set to correspond to the level of the culture medium in the lower chamber.

4. A method of culturing 3D cell aggregate using a 3D culture device according to one of the claims 1 to 3, the method comprises steps of:

   seeding cells on the porous membrane of the upper chamber;
   introducing a cell culture medium into the upper portion of the upper chamber through an opening; and
   discharging the cell culture medium, that permeated the porous microwell in the upper chamber and flowed into the lower chamber, from the lower chamber.

5. The method according to claim 4, wherein the cell is at least one selected from the group consisting of stem cells including induced pluripotent stem cells and mesenchymal stem cells; cell lines including myoblasts, embryo fibroblasts, umbilical vein endothelial cells, liver cancer cells (HepG2 cells), and epidermal cells; and primary cells obtained from liver, pancreas, or small intestine.

6. The method according to claim 4, further comprises a step of introducing the cell culture medium into the lower chamber.

Fig. 1

Fig. 2

Fig. 3

31

30

(a)

31

30

(b)

Fig. 4

10

30

23　21　22　23

Fig. 5

Fig. 6

(a)

10

20

40

21

(b)

10

20

21

40

(c)

10

20

40

21

Fig. 7

| Preparation step |～S10, S100 |
| Formation step |～S20, S200 |

Fig. 8

(a)

(b)

(c)

Fig. 9

Fig. 10

(a)

(b)

(c)

(d)

1mm

■ 22     ■ 21

(e)

(f)

Fig. 11a

Fig. 11b

Fig. 12

Fig. 13a

Fig. 13b

Fig. 14a

Fig. 14b

Fig. 14c

Fig. 15a

Opening(11)

Porous microwell(21)

Upper chamber(100)

Bottom flow

Fluid outlet(330)

Fluid inlet(310)

Lower chamber(200)

Fig.15b

Opening(11)

Porous microwell(21)

Through portion(400)

Upper chamber(100)

Fluid outlet(330)

Fluid inlet(310)

Bottom flow

Lower chamber(200)

Fig. 15c

Fig. 16a

Fig. 16b

Fig. 17

Fig. 18a

Fig. 18b

Culture medium inflow     Culture medium discharge

~Upper chamber

Culture medium

Nanofibrous microwell
Permeating micro-flow

~Lower chamber

Fig. 19a

Culture medium inflow

Storage of discharged culture medium

Culture medium discharge

Fig. 19b

Porous microwell(21)

Upper chamber(110)

Lower chamber(210)

Fig. 19c

Culture medium inflow

Inlet(311)

Upper chamber(110)

Porous microwell(21)

Culture medium reservoir(65)

Lower chamber(210)

Outlet(331)

Fig. 20a

sink

Fig. 20b

Fig. 20c

Fig. 21

(a)　　　　　　　　　　(b)

a) top view　　b) Enlarged top view　　c) Enlarged side view

(c)

Fig. 22a

Fig. 22b

Fig. 22c

Fig. 22d

Fig. 23

Fig. 24

Fig. 25

Blue solution: Cell culture medium used
Transparent solution: Fresh cell culture medium

Fig. 26

Fig. 27a

Fig. 27b

Fig. 28

Compression process

Convex mold

Concave mold

Fig. 29a

Fluid discharge

Fig. 29b

Fluid discharge

Fig. 29c

## INTERNATIONAL SEARCH REPORT

| International application No. |
| --- |
| **PCT/KR2022/006037** |

**A.    CLASSIFICATION OF SUBJECT MATTER**

**C12M 3/00**(2006.01)i; **C12M 1/32**(2006.01)i; **C12M 1/12**(2006.01)i

According to International Patent Classification (IPC) or to both national classification and IPC

**B.    FIELDS SEARCHED**

Minimum documentation searched (classification system followed by classification symbols)

C12M 3/00(2006.01); B29C 51/08(2006.01); C12M 1/00(2006.01); C12M 1/12(2006.01); C12M 1/32(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다공성 마이크로웰(porous microwell), 배양(culture), 세포(cell), 챔버(chamber), 유체(fluid), 유동(flow), 마이크로웰(microwell), 투과(penetration), 배출구(outlet)

**C.    DOCUMENTS CONSIDERED TO BE RELEVANT**

| Category* | Citation of document, with indication, where appropriate, of the relevant passages | Relevant to claim No. |
| --- | --- | --- |
| X | KR 10-2020-0081853 A (POSTECH RESEARCH AND BUSINESS DEVELOPMENT FOUNDATION) 08 July 2020 (2020-07-08)<br>      See claim 1; paragraphs [0005], [0010] and [0061]-[0062]; and figures 1 and 5. | 1-2,4-6 |
| Y | | 3 |
| Y | KR 10-2019-0115604 A (KOREA INSTITUTE OF INDUSTRIAL TECHNOLOGY) 14 October 2019 (2019-10-14)<br>      See paragraphs [0041]-[0042] and [0067]; and figure 5. | 3 |
| A | KR 10-2018-0091763 A (POSTECH RESEARCH AND BUSINESS DEVELOPMENT FOUNDATION) 16 August 2018 (2018-08-16)<br>      See entire document. | 1-6 |
| A | KR 10-2019-0121544 A (POSCO et al.) 28 October 2019 (2019-10-28)<br>      See entire document. | 1-6 |

☑ Further documents are listed in the continuation of Box C.          ☑ See patent family annex.

| | |
| --- | --- |
| *     Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **04 August 2022** | **04 August 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/006037** |

| C. | DOCUMENTS CONSIDERED TO BE RELEVANT | |
| --- | --- | --- |
| Category* | Citation of document, with indication, where appropriate, of the relevant passages | Relevant to claim No. |
| A | KR 10-2020-0071966 A (T&R BIOFAB CO., LTD.) 22 June 2020 (2020-06-22)<br>  See entire document. | 1-6 |
| PX | KR 10-2021-0098266 A (POSTECH RESEARCH AND BUSINESS DEVELOPMENT FOUNDATION)<br>10 August 2021 (2021-08-10)<br>  See claims 1-7; paragraph [0026]; and figure 1. | 1-6 |

Form PCT/ISA/210 (second sheet) (July 2019)

**INTERNATIONAL SEARCH REPORT**
Information on patent family members

International application No.

**PCT/KR2022/006037**

| Patent document cited in search report | | | Publication date (day/month/year) | Patent family member(s) | | | Publication date (day/month/year) |
|---|---|---|---|---|---|---|---|
| KR | 10-2020-0081853 | A | 08 July 2020 | KR | 10-2224065 | B9 | 05 March 2021 |
| | | | | US | 2022-0073852 | A1 | 10 March 2022 |
| | | | | WO | 2020-138581 | A1 | 02 July 2020 |
| KR | 10-2019-0115604 | A | 14 October 2019 | KR | 10-2064769 | B1 | 13 January 2020 |
| KR | 10-2018-0091763 | A | 16 August 2018 | | None | | |
| KR | 10-2019-0121544 | A | 28 October 2019 | KR | 10-2068465 | B1 | 21 January 2020 |
| KR | 10-2020-0071966 | A | 22 June 2020 | | None | | |
| KR | 10-2021-0098266 | A | 10 August 2021 | KR | 10-2022-0024276 | A | 03 March 2022 |
| | | | | WO | 2021-153836 | A1 | 05 August 2021 |

Form PCT/ISA/210 (patent family annex) (July 2019)